# EUROPÄISCHE PATENTSCHRIFT

(11) **EP 1 933 691 B1**
(45) Veröffentlichungstag und Bekanntmachung des Hinweises auf die Patenterteilung: **14.01.2015**
(21) Anmeldenummer: 06807135.6
(22) Anmeldetag: 11.10.2006
(51) Int. Cl.: A61B 1/01

(54) **MEDIZINTECHNISCHE VORRICHTUNG MIT SELBSTSCHMIERENDEM ELEMENT**
TECHNICAL MEDICAL DEVICE COMPRISING A SELF-LUBRICATING ELEMENT
DISPOSITIF MEDICAL DOTE D'UN ELEMENT AUTOLUBRIFIANT

(30) Priorität: 11.10.2005 DE 102005048675
(43) Veröffentlichungstag der Anmeldung: 25.06.2008
(73) Patentinhaber: INVENDO MEDICAL GMBH, 69469 Weinheim (DE)
(72) Erfinder: BOB, Konstantin, 69469 Weinheim (DE)
(74) Vertreter: Winter, Brandl, Fürniss, Hübner, Röss, Kaiser, Polte - Partnerschaft mbB
(86) Internationale Anmeldenummer: PCT/EP2006/067258
(87) Internationale Veröffentlichungsnummer: WO 2007/042526

(56) Entgegenhaltungen:
- WO-A-01/90230
- WO-A-89/09246
- WO-A-2005/058139
- DE-U1-202005 004 168
- US-A- 5 236 423
- US-A- 5 674 219
- US-A1- 2002 147 385
- US-A1- 2003 229 269
- US-B1- 6 257 238

## Beschreibung

### GEBIET DER ERFINDUNG

Die vorliegende Erfindung bezieht sich auf eine medizintechnische Vorrichtung, die mit einem menschlichen oder tierischen Körper in Eingriff gebracht werden kann.

### HINTERGRUND DER ERFINDUNG

Medizintechnische Vorrichtungen, welche mit einem menschlichen oder tierischen Körper in Eingriff gebracht werden können, sind vielfältig. Beispiele für solche Vorrichtungen schließen etwa Endoskope, Katheter, Nadeln, usw. ein.

Endoskopievorrichtungen sowie Vorrichtungen zum Einführen eines medizinischen Endoskops in einen Körperkanal werden beispielsweise in der offengelegten deutschen Patentanmeldung DE-A-39 25 484 beschrieben. Die darin beschriebenen Vorrichtungen erlauben es, dass ein Endoskop nicht mehr in den zu untersuchenden Körper hineingeschoben wird, sondern sich selber hineinbewegt. Zu diesem Zweck ist das Endoskop mit einem Eigenantrieb ausgestattet, der ein unkomplizierteres und schnelleres Einführen ermöglicht.

Als solch ein Eigenantrieb kann beispielsweise auch ein so genannter Stülpschlauch verwendet werden, in den der Endoskopschaft eingeführt ist. Beim Vortrieb des.Endoskops treten unterschiedliche Relativbewegungen auf. Zum einen tritt eine Relativbewegung zwischen dem Endoskopschaft und dem Stülpschlauch auf, die miteinander in Gleitkontakt stehen. Zum anderen tritt auch eine Relativbewegung zwischen einem innenliegenden und einem außenliegenden Abschnitt des sich abwickelnden Stülpschlauchs auf.

Um die jeweils auftretende Gleitreibung zu verringern, ist beispielsweise der Einsatz eines von Außen zugeführten Schmiermittels vorgeschlagen worden, beispielsweise in EP-A-O 873 761. Die Figuren 1 und 2 zeigen Beispiele der in EP-A-O 873 761 beschriebenen Endoskopievorrichtungen. In beiden Fällen wird zwischen den Endoskopschaft 1 und den Stülpschlauch 2 einerseits sowie zwischen die beiden übereinander zu liegen kommenden Stülpschlauchabschnitte andererseits ein Schmiermittel zugeführt. Dieses Schmiermittel wird in äußeren Reservoirs 3 und 4 bevorratet und bei der Verwendung eingepumpt.

Schließlich offenbart die US 5,236,423 ein Endoskop bestehend aus einem Endoskopschaft und einem diesen umgebenden Stülpschlauch als Schaftantrieb. Der Ringraum zwischen Schaft und Schlauch ist mit einem druckbeaufschlagten Schmiermittel angefüllt. Der Schaft hat eine glatte Oberfläche. Der Schlauch kann u. a. auch aus Silikongummi hergestellt sein, wohingegen das Schmiermittel PVP, Wasser, Glycerin oder mineralisches/pflanzliches Öl enthalten kann.

### ZUSAMMENFASSUNG DER ERFINDUNG

Eine Aufgabe der vorliegenden Erfindung besteht in der Bereitstellung einer verbesserten medizintechnischen Vorrichtung, insbesondere einer verbesserten Endoskopievorrichtung. Spezielle Verbesserungen, die vorzugsweise erzielt werden sollen, sind insbesondere eine Vereinfachung des Aufbaus der medizintechnischen Vorrichtung, erhöhte Wirtschaftlichkeit, verbesserte Akzeptanz beim Individuum, bei dem die Vorrichtung zum Einsatz kommt, sowie eine Verringerung der Reibungskräfte, die in der medizintechnischen Vorrichtung auftreten.

Erfindungsgemäß wird die Aufgabe durch eine mit einem menschlichen oder tierischen Körper in Eingriff bringbare medizintechnische Vorrichtung mit einem einer inneren Reibung in der Vorrichtung ausgesetzten selbstschmierenden Element mit den Merkmalen des Anspruchs 1 gelöst.

In einer bevorzugten Ausführungsform ist es das selbstschmierende Element selber, welches mit einem menschlichen oder tierischen Körper in Eingriff bringbar ist und mit dem Körper in Kontakt tritt.

Weiter ist es bevorzugt, dass mindestes eine Oberfläche des selbstschmierenden Elements nämlich des Endoskopschafts plasmabehandelt ist, wobei die plasmabehandelte Oberfläche mehr bevorzugt plasmabeschichtet ist.

Vorteilhafterweise ist dabei die plasmabeschichtete Oberfläche mit Siloxan, Fluorcarbon, PTFE, ePTFE und/oder Polyurethan beschichtet.

Wenigstens eine Oberfläche des selbstschmierenden Elements ist bevorzugt oberflächenbehandelt, beispielsweise durch UV-Bestrahlung oder Silicatisierung.

Das selbstschmierende Element der erfindungsgemäßen Vorrichtung kann bevorzugt eine Keramikbeschichtung, eine Acrylatbeschichtung und/oder eine Arylbeschichtung aufweisen.

Erfindungsgemäß ist es für den Stülpschlauch vorgesehen, dass er Silikonkautschuk aufweist, weiter bevorzugt aus Siliconkautschuk besteht.

Außerdem weist der Stülpschlauch eine Oberflächenschicht auf, welche ein Schmiermaterial, insbesondere ein Material ausgewählt aus der Gruppe bestehend aus Graphit, Nitrid, Fluorkohlenstoff, MOS2, WS2, Talk, Siliconöl, Polysilazan, Polysiloxan, PTFE, ePTFE, Gelatine, Agar-Agar und Cellulose, eine Kombination aus einem Polysaccharid und einem Protein oder ein Siliconöl, z.B. ein hydrophiles

Siliconol oder ein hydrophobes Siliconol, aufweist.

In einer bevorzugten Ausgestaltung dieser Ausführungsform ist die Oberflache des selbstschmierenden Elements hydrophil, in einer anderen bevorzugten Ausgestaltung hydrophob. Hydrophile Oberflächen haben gegenüber Wasser Kontaktwinkel, die kleiner sind als 90[deg.], hydrophobe Oberflachen hingegen von 90[deg.] oder mehr. Kontaktwinkel meint dabei den Winkel, den eine Tangente an die Tropfenkontur im Drei-Phasen-Punkt (festflüssig-gasförmig) zur Oberfläche des Festkörpers (der Oberflache) bildet und somit ein Maß für die Benetzbarkeit einer Oberflache oder Grenzflache durch eine andere Phase darstellt. Je größer der Kontaktwinkel, umso geringer ist die Benetzung.

Gemäß der Erfindung weist wenigstens eine Oberfläche des selbstschmierenden Elements in Form des Endoskopschafts Kavitäten auf, die ein Schmiermittel enthalten. Dabei ist es bevorzugt, dass das Profil durch Gravieren mit einem Laser erzeugbar ist.

Dabei ist es bevorzugt, wenn über den Kavitäten eine Plasmabeschichtung ausgebildet ist.

Ferner sind die Kavitäten bevorzugt so angepasst, dass freigesetztes Schmiermittel zu Bereichen des selbstschmierenden Elements zugeführt wird, welche einer größeren Reibung ausgesetzt sind als andere Bereiche.

Insbesondere ist es bevorzugt, dass die Kavitäten so angepasstsind, dass bei Überlagerung zweier entgegenstehender Abschnitte des selbstschmierenden Elements die jeweiligen Kavitätenabschnitte nicht ineinander greifen können.

In einer weiteren Ausführungsform der Erfindung besteht das selbstschmierende Element in Form des Stülpschlauchs aus einem Werkstoff, in dem ein Schmiermittel nahezu homogen verteilt ist. Hierbei ist es weiter bevorzugt, dass zwischen Schmiermittel und Werkstoff eine Unverträglichkeit besteht, so dass es zu einer fortschreitenden Trennung von Schmiermittel und Werkstoff kommt.

Außerdem ist es bevorzugt, dass das selbstschmierende Element porös ist.

Bezüglich des Schmiermittels ist es bevorzugt und vorteilhaft, wenn die Temperatur-Viskositäts-Charakteristik des Schmiermittels im Temperaturbereich von 10-43<0>C eine Unstetigkeit aufweist.

Gemäß der Erfindung wird des Weiteren ein Verfahren zur Herstellung einer medizintechnischen Vorrichtung bereitgestellt, die mit einem menschlichen oder tierischen Körper in Eingriff bringbar ist und ein selbstschmierendes Element aufweist, wobei das Verfahren einen Schritt aufweist, bei dem ein nicht selbstschmierendes Element so modifiziert wird, dass dieses zu dem selbstschmierenden Element wird.

### BESCHREIBUNG DER FIGUREN

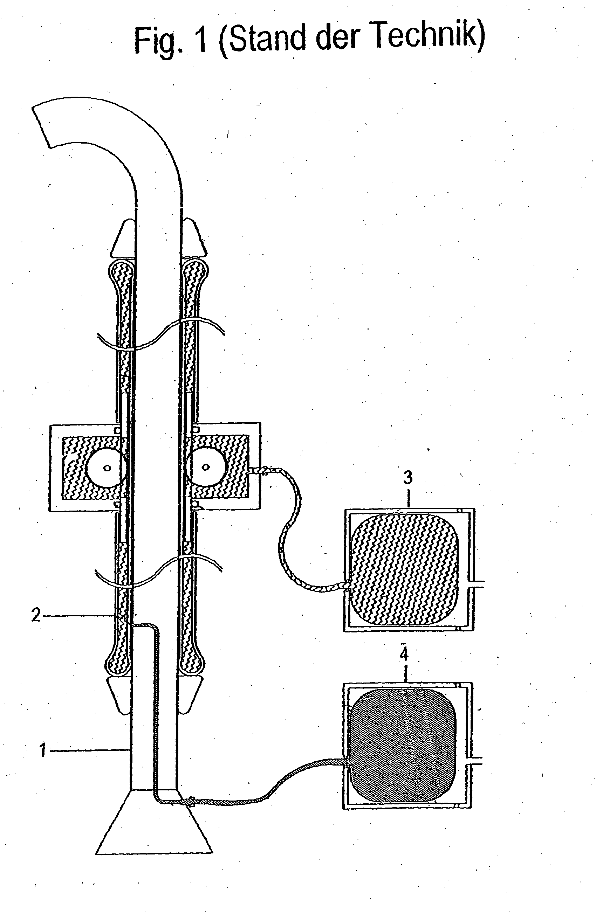

Die Fig. 1 veranschaulicht eine Endoskopievorrichtung des Stands der Technik.

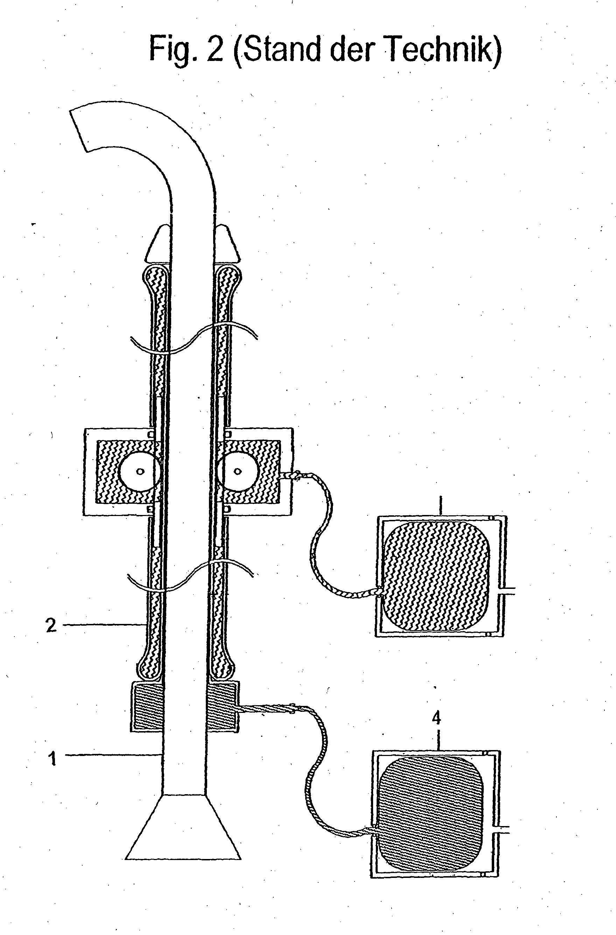

Die Fig. 2 veranschaulicht eine weitere Endoskopievorrichtung des Stands der Technik.

### BEVORZUGTE AUSFUHRUNGSFORMEN DER ERFINDUNG

Die Erfindung stellt eine mit einem menschlichen oder tierischen Körper in Eingriff bringbare medizintechnische Vorrichtung mit einem einer inneren Reibung in der Vorrichtung ausgesetzten selbstschmierenden Element bereit.

Ein selbstschmierendes Element gemäß der vorliegenden Erfindung ist ein Element, das ohne Zuführung eines Schmiermittels von Außen eine Schmierwirkung entfalten kann. Unter Schmierwirkung wird dabei insbesondere die Herabsetzung des Reibungskoeffizienten bei einer Reibung zwischen dem selbstschmierenden Element und einem Gegenelement, wobei letzteres mit dem selbstschmierenden Element in Kontakt steht, sowie bei einer Reibung zwischen unterschiedlichen Abschnitten des selbstschmierenden Elements verstanden.

Die selbstschmierenden Eigenschaften ermöglichen eine signifikante Verringerung der inneren Reibung, insbesondere der Reibung zwischen unterschiedlichen Abschnitten des selbstschmierenden Elements sowie zwischen dem Element einerseits und anderen Teilen der medizintechnischen Vorrichtung andererseits.

Zudem ergibt sich im Gegensatz zu einer äußeren Zufuhr von Schmiermittel eine gerätetechnische Vereinfachung und eine deutlich verbesserte Handhabbarkeit der medizintechnischen Vorrichtung. Die selbstschmierenden Eigenschaften des Elements in der Vorrichtung an sich machen es ferner möglich, die Vorrichtung als einfacher herzustellenden und billigeren Einwegartikel auszugestalten. Darüber hinaus wird der Verbrauch an Schmiermittel verringert, was weitere wirtschaftliche Vorteile hat.

Auch das im Stand der Technik auftretende Phänomen, dass Schmiermittel in den untersuchten menschlichen oder tierischen Körper gelangt, kann durch die erfindungsgemäße Vorrichtung weitestgehend bzw. ganz verhindert werden.

Als besonders gute Materialien für das selbstschmierende Element kommen insbesondere unvulkanisierter oder vulkanisierter Siliconkautschuk, PTFE, ePTFE und Polyurethan(e) in Betracht, da diese gut verarbeitbar sind, bereits als solche selbstschmierende Eigenschaften aufweisen können und sehr gute mechanische Eigenschaften wie Schlagzähigkeit, Biegeverhalten, etc. haben, die insbesondere bei einer Verwendung des selbstschmierenden Elements als Schlauch, insbesondere als Stülpschlauch, in geeignetem Ausgleich zueinander stehen. Die genannten Materialien können mit weiteren Materialien vermengt sein.

Gemäß einer bevorzugten Ausführungsform der Erfindung umfasst der Werkstoff, welcher einen wesentlichen Bestandteil des selbstschmierenden Elements bildet, einen oder mehrere Kautschuke ausgewählt aus der Gruppe bestehend aus Siliconkautschuken, natürlichen Kautschuken und (Co)polymerkautschuken.

Als (Co)polymerkautschuke sind Kautschuke aus Copolymeren geeignet, die mindestens ein, üblicherweise zwei, drei oder mehr unterschiedliche Monomere umfassen.

Des Weiteren können Kautschukzusammensetzungen der vorliegenden Erfindung geeignete Weichmacher, Füllstoffe und/oder Schaumbildner und dergleichen erhalten.

Siliconkautschuke sind als Grundsubstanzen des erfindungsgemäßen Kautschukelements besonders vorteilhaft einsetzbar und daher bevorzugt.

Bei Verwendung eines Siliconkautschuks kann das Kautschukelement gegebenenfalls zusätzlich zu den vorstehend beschriebenen Zusatzstoffen

Hydrophobierungsmittel enthalten. Diese verhindern eine Aufnahme von Wasser und in der Folge, dass aufgenommenes Wasser zu einer Auftrennung von Kettenelementen und damit zu unerwünschten Veränderungen des Siliconkautschuks führt.

Als Hydrophobierungsmittel lassen sich beispielsweise silanolhaltige Siliconverbindungen und/oder Polyalkyldisilazane verwenden.

Als weitere Hilfsstoffe können zusätzlich Rhodium- und/oder Platinkatalysator(en) und gegebenenfalls ein oder mehrere Inhibitoren zugegeben sein. Für eine Additionsvernetzung sind insbesondere Platinkatalysatoren geeignet, vorzugsweise Pt(0)-Komplexe, die als Liganden z.B. Vinylsiloxane aufweise können.

Für mögliche Inhibitoren lassen sich speziell acetylenische Alkohole wie z.B. 2-Methylbutinol und Ethinylcyclohexanol, Tetramethyltetravinylcyclotetrasiloxan oder Tetramethyldivinyldisiloxan angeben.

Wird der Siliconkautschuk peroxidisch vernetzt (vulkanisiert), so können anstatt des Katalysators vorzugsweise Alkyl- oder Arylperoxide wie z.B. Dicumylperoxid, 1,4-Bis(tert-butylperoxy)-1,4-dimethylhexan, 2,4-Dichlorbenzoylperoxid und 4-Methylbenzoylperoxid zugegeben sein.

Ferner sind für medizintechnische Anwendungen auch halogenhaltige, insbesondere fluorhaltige Polyolefine geeignet, da sie oftmals inert sind und dementsprechend eine sehr gute Körperverträglichkeit aufweisen. Aufgrund seiner inerten und biokompatiblen Eigenschaften und der leichten Zugänglichkeit ist dabei PTFE am meisten bevorzugt, entweder als normales PTFE oder in expandierter Form (ePTFE).

Vorteilhafter Weise ist das selbstschmierende Element durch eine Oberflächenbehandlung eines unbehandelten Elements erhältlich, um seine selbstschmierenden Eigenschaften auszubilden bzw. zu verstärken. Geeignete Verfahren zur Oberflächenbehandlung sind beispielsweise Silicatisierung, UV-Bestrahlung, Ozonbehandlung, Texturieren mittels Fotolack- oder Siebdrucktechniken und eine Corona-Behandlung.

Auch ist es möglich, gezielt weitere Schichten aufzubauen, etwa durch Schleuder-, Eintauch- oder Sprühbeschichtung, evtl. Polymerisation einer aufgebrachten Lösung mittels e⁻- oder γ-Strahlen, Pfropfpolymerisation durch Fotokopplung, Keramikbeschichtung, Acrylatbeschichtung, Polysilazanbeschichtung, Polysiloxanbeschichtung und Arylbeschichtung. Geeignete Verfahren zum Auftragen von Beschichtungen zur Oberflächenbehandlung sind in diesem Zusammenhang u.a. auch Sputtern, PVD (physical vapor deposition) und CVD (chemical vapor deposition) sowie eine Pulverbeschichtung. ,

Als ein weiteres Verfahren ist die Plasmabehandlung anzusprechen, welche vielfältige Möglichkeiten der Oberflächenbehandlung ermöglicht, z.B. in Abhängigkeit vom zu behandelnden Material, und besonders gute selbstschmierende Eigenschaften hervorbringen kann. Zudem ist die Plasmabehandlung wirtschaftlich sehr interessant, da große Stückzahlen zu geringen Kosten in vergleichsweise kurzer Zeit hergestellt werden können.

Unter Plasma versteht man ein ionisiertes Gas mit gleicher Anzahl positiver und negativer Ladungen. Typischer Weise eingesetzte Gase sind hierbei insbesondere O₂ und F₂ als Ätzgase, mit denen eine Reinigung der Oberfläche des Werkstücks vorgenommen werden kann; Edelgase wie Argon sowie Wasserstoff, welche über UV-Wirkung sowie Fragmentierung und erneute Abscheidung eine Vernetzung hervorrufen können; NH₃, N₂, SO₂, Luft und Wasser, welche eine Funktionalisierung und damit Aktivierung der Oberfläche ermöglichen; organische und metallorganische Gase, welche durch Abscheidung eine Plasmapolymerschicht ausbilden können; sowie Halogengase; und Gemische aus den vorstehend genannten Gasen.

Mit allen genannten Gasen lassen sich Radikalstellen erzeugen, welche weitere Behandlungen wie etwa eine Nachvernetzung, Nachoxidation und Pfropfung mit funktionellen Monomeren ermöglichen, die sich im Endprodukt als Polymerschicht wiederfinden.

Durch die Auswahl der eingesetzten Gase können zudem ganz bestimmte Funktionalisierungen der behandelten Oberfläche erzielt werden. Beispielsweise kann eine =O-Gruppe mittels O₂ eingeführt werden, eine OH-Gruppe mittels H₂O, H₂/O₂ oder O₂, eine COOH-Gruppe mittels H₂O, H₂/O₂ oder CO₂/H₂, eine F-Gruppe mittel CF₄, SF₆, BF₃, XeF₆, SOF₂, NF₃ oder SiF₄, eine Cl-Gruppe mittels, CCl₄ oder BCl₃, eine NH₂-Gruppe mittels NH₃, N₂H₄ oder N₂/H₂ und eine SH-Gruppe mittels H₂S, H₂/S₈ oder CS₂.

Durch Anlegen einer hohen Frequenz können diese Gase in ihre entsprechenden Plasmen überführt werden, die dann üblicher Weise Elektronen, Radikale, Ionen, UV-Strahlung und, in Abhängigkeit vom eingesetzten Gas, eine Vielzahl unterschiedlicher angeregter Teilchen enthalten.

Bei der Oberflächenbehandlung kommen in der Erfindung u.a. Plasmen mit geringem Druck zum Einsatz, z.B. mit einem Druck von 1 bis 1000 Pa, was die Ausnutzung des chemisch extrem reaktiven Plasmas zur Oberflächenbehandlung vereinfacht. Geeignete Leistungen zum Zünden des Plasma, z.B. durch Glimmentladung, liegen im Bereich von bevorzugt 10 bis 5000 W. Typische Behandlungszeiten reichen von einigen Sekunden bis hin zu mehreren Minuten.

Die eigentliche Oberflächenbehandlung kann dabei direkt in der Plasmazone, außerhalb der Plasmazone (so genanntes "remote-Verfahren") oder zunächst zur Aktivierung in der Plasmazone mit anschließender Propfreaktion erfolgen. Es ist auch möglich, das zu behandelnde Element zunächst mit einer Reaktionslösung, insbesondere einer Polymerlösung, beispielsweise durch Eintauchen zu beschichten und anschließend einen Fixierschritt innerhalb der Plasmazone durchzuführen.

Durch die Plasmabehandlung kann die Oberfläche des Elements auf unterschiedliche Weise modifiziert werden. Eine erste Modifizierung besteht in einer Reinigung und Sterilisation durch Ätzabtrag. Bei diesem Ätzvorgang wird die Benetzbarkeit der Oberfläche verändert und insbesondere deren Rauhigkeit vergrößert. Konkret wird durch Materialabtrag die Mikrorauhigkeit der Oberfläche vergrößert, d.h. eine raue Textur mit Unebenheiten erzeugt, deren durchschnittliche Tiefe, gemessen vom Wellenberg bis zum Wellental, im Bereich von bevorzugt 1 bis 2500 nm, mehr bevorzugt 1 bis 1500 und am meisten bevorzugt 1 bis 1000 nm liegt. Hierbei handelt es sich also um eine Plasmavorbehandlung oder -aktivierung, an welche sich weitere Schritte anschließen, um letztlich zum selbstschmierenden Element der Erfindung zu gelangen. Es hat sich gezeigt, dass ein selbstschmierendes Element gemäß der Erfindung, insbesondere aus Siliconkautschuk, eine verbesserte selbstschmierende Eigenschaft zeigt, wenn es zunächst die Plasmaaktivierung durchlaufen hat.

Die erzeugte Mikrorauhigkeit erleichtert wesentlich die Auftragung weiterer Substanzen bzw. Schichten, insbesondere von weiteren Schmiermitteln gemäß der vorliegenden Erfindung. In einer bevorzugten Ausführungsform wird dabei als Schmiermittel Siliconöl, insbesondere hydrophiliertes Siliconöl, aufgetragen, welches in einem sich anschließen, zweiten Plasmaverfahren auf der vorbehandelten Oberfläche fixiert wird. Die Dicke der aufgetragenen Siliconölschicht liegt dabei bevorzugt im Bereich von 0,1 bis 1 nm.

Eine zweite Modifizierung besteht in einer Erzeugung von Radikalstellen. Durch die Ausbildung dieser Radikalstellen kann eine Sekundärreaktion befördert werden, z.B. eine weitergehende Vernetzung einer aufgetragenen, insbesondere polymeren Substanz. Auf diese Weise kann eine dichtere Ausbildung des Polymernetzwerks erfolgen, was insgesamt die Schmiereigenschaften der resultierenden Oberfläche verbessert.

Diese Wirkung wird weiter verstärkt, wenn die Radikalstellen Ausgangspunkt für eine sich anschließende Pfropfreaktion sind. Dabei wird auf die bereits behandelte Oberfläche eine polymere Substanz aufgepfropft, welche vorzugsweise von dem Grundmaterial für das selbstschmierende Element (z.B. Siliconkautschuk oder PTFE) verschieden ist. Auf diese Weise lassen sich beispielsweise Vinylpyrrolidon, Vinylimidazol, Ethoxid, (Block)Copolymere aus Polyethylenoxid und Polypropylenoxid, allylisches Polyethylenoxid und (2-Hydroxyethyl)methacrylat aufpfropfen.

Die ausgebildeten Radikalstellen sind des Weiteren dazu geeignet, durch Reaktion mit z.B. Sauerstoff, etwa dem Luftsauerstoff, hydrophile Stellen auszubilden, so dass die behandelte Oberfläche insgesamt hydrophil gemacht wird.

Erfindungsgemäß kommt bevorzugt die so genannte Niederdruckplasmabehandlung zum Einsatz. Niederdruckplasmaverfahren bedeutet, dass in einer Vakuumkammer, in der ein sehr geringes Vakuum von üblicherweise unter 0,5 mbar existiert, die Schläuche eingelegt werden, um anschließend bei einer Temperatur von beispielsweise 20-40°C durch Ionisation mit ca. 1.000 Volt mit z.B. Siloxan oder Polycarbonen beschichtet zu werden. Durch die Gaseinleitung entstehen auf die oben beschriebene Weise Radikale an der Oberfläche der Schläuche und aus monomeren Silanmolekülen entstehen Polymere (Polymerisation).

Im Falle eines schlauchförmigen Arbeitsstücks kann hierbei auf sehr einfache Weise eine Außen- und Innenbeschichtung erfolgen. Dies kann in einem Arbeitsgang erfolgen, indem zusätzlich zu der oben erwähnten Beschichtung eine/zwei Elektroden an das/die Ende(n) der.Schläuche angelegt wird/werden, so dass diese Elektrode(n) dafür sorgt/sorgen, dass auch die Innenwandung der Schläuche beschichtet wird.

Es gibt zusätzlich zu der Niederdruckplasmabehandlung noch eine so genannte Atmosphärendruckplasmabehandlung, die erfindungsgemäß ebenfalls eingesetzt werden kann.

Zudem unterscheidet man die eingesetzten Plasmen in Abhängigkeit von der eingesetzten Temperatur, vornehmlich in ein Niedertemperaturplasma und ein Hochtemperaturplasma. Das Hochtemperaturplasma arbeitet mit Fusionsplasmen, wobei die Gastemperatur typischerweise im Bereich von 10⁷ K liegt. Das Niedertemperaturplasma wird weitergehend in ein nicht-thermisches sowie ein thermisches Plasma unterteilt.

Das nicht-thermische Plasma wird z.B. durch NiederdruckGlimmentladung, Korona-Entladung oder Barriere-Entladung erzeugt, wobei die Gastemperatur typischerweise im Bereich von 300 bis 10³ K liegt. Das thermische Plasma wird z.B. durch Bogenentladung, Bogenstrahlplasma oder eine RF-Bogenfackel erzeugt, wobei die Gastemperatur typischerweise nicht höher als 2*10⁴ K liegt.

Die für die Beschichtung erforderliche Zeit kann in Abhängigkeit vom Material des Werkstücks, der aufzubauenden Schicht und deren Dicke geeignet eingestellt werden und liegt typischerweise im Minutenbereich, wobei auch Behandlungszeiten von bis zu einer halben Stunde in betracht kommen.

Erfindungsgemäß werden die folgenden Ionisationsverfahren bevorzugt eingesetzt: In einem Verfahren wird Sauerstoff als Gas eingesetzt, um ein vorgelegtes Siliconmaterial so verändern, dass eine glatte Oberfläche resultiert. In einem weiteren Verfahren wird Siloxan über eine flüssige Vorform in einen gasförmigen Zustand umgewandelt, dieselbe Vorgehensweise ist für eine Beschichtung mit Fluorcarbon bevorzugt. Die beiden letzten Varianten haben der Vorteil einer noch glatteren Oberfläche, was eine deutliche Reduktion der Reibekräfte zur Folge hat, wobei noch bessere Ergebnisse erzielt werden, wenn die beiden Verfahren nacheinander durchgeführt werden.

Aus diesem Grund sind durch Plasmabehandlung erhältliche, mit Siloxan und/oder Fluorcarbon beschichtete Siliconschläuche besonders bevorzugte Ausführungsformen der vorliegenden Erfindung. Daneben ist es für eine verbesserte Gleiteigenschaft zudem bevorzugt, durch eine Plasmabeschichtung eine PTFE- oder ePTFE-Schicht auf dem selbstschmierenden Element aufzubauen, insbesondere auf einem selbstschmierenden Siliconelement wie etwa einem Siliconschlauch. Durch diese Be'schichtung mit (e)PTFE können weitere Verminderungen des Reibungskoeffizienten des selbstschmierenden Elements erzielt werden.

Ferner ist es durch die vorstehend erwähnte Plasmapolymerisation (vorausgehendes Auftragen von Polymerlösung gefolgt von Fixieren im Plasma) möglich, weitere Schichten mit erwünschten Eigenschaften aufzubauen, die ihrerseits zur Selbstschmierung beitragen können. Bei diesem Schichtaufbau kann wiederum ausgewählt werden, ob die Oberfläche hydrophile oder hydrophobe Eigenschaften aufweisen soll.

Es ist ganz grundsätzlich festzuhalten, dass durch die Oberflächenbehandlung eine anwendungsabhängige gezielte Einstellung der Oberfläche des selbstschmierenden Elements der Erfindung erzielt werden kann. D.h., die Oberflächenenergie kann in Abhängigkeit davon, ob noch weitere Schichten und/oder Schmiermittel aufzutragen sind, ganz gezielt eingeregelt werden.

Chemisch ist dabei vorteilhaft, dass die Oberfläche gezielt mit hydrophilen oder hydrophoben Eigenschaften ausgestattet werden kann, was zu einer verbesserten Schmierwirkung führt, wenn zwei unterschiedliche Abschnitte des Elements miteinander in Gleitkontakt treten, und zudem die Auftragung weiterer Beschichtungen in Abhängigkeit von deren Hydrophilie oder -phobie erleichtert. Zudem ist auch eine gezielte Aktivierung der Oberfläche durch Einführen aktiver Gruppen auf diese Weise zugänglich. Um eine hydrophobe Oberfläche zu erhalten, werden bevorzugt Siloxanplasmen eingesetzt, insbesondere Siloxan-Perflüorcarbonplasmen. Um ölabstoßende Eigenschaften zu erzielen, kommt bevorzugt eine Pfropfung mit Acrylaten, insbesondere mit Perfluoracrylaten in Betracht.

Die Einstellung der Oberfläche auf eine hydrophile oder hydrophobe Oberfläche ist insbesondere von einem optional ergänzend einzusetzenden Schmiermittel abhängig. Beispielsweise kann, wenn die Oberfläche des selbstschmierenden Elements hydrophil ausgebildet ist, als ergänzendes Schmiermittel Wasser oder eine wässrige Lösung bzw. eine wässrige Mischung eingesetzt werden. Gerade bei endoskopischen Anwendungen, in denen herkömmlich oftmals ölige Gleitmittel eingesetzt werden, welche vom untersuchten Individuum weniger gut vertragen oder akzeptiert werden, führt der Einsatz von Wasser oder wässrigen Lösungen/Mischungen zu deutlichen Verbesserungen.

Ist es andererseits erforderlich oder erwünscht, ergänzend ölige Schmiermittel einzusetzen, besteht eine besonders gute Kompatibilität mit dem selbstschmierenden Element und ergibt sich damit eine verbesserte Gleitwirkung, wenn die Oberfläche des selbstschmierenden Elements hydrophob gemacht ist.

Eine vorteilhafte Beschichtung (Coating) auf der Oberfläche des Elements vermindert bereits als solche die Reibungseigenschaften signifikant und trägt somit zu den selbstschmierende Eigenschaften des beschichteten Elements bei. Um zum Aufbringen geeignete Oberflächeneigenschaften zu gewährleisten, kann es vorteilhaft sein, das Element zunächst einer chemischen, mechanischen oder physikalischen Vorbehandlung zu unterziehen, insbesondere einer Coronavorbehandlung.

Geeignete Eigen- bzw. Selbstschmierung entwickelnde Beschichtungsmaterialien sind insbesondere Graphit, Nitride wie Bornitrid, Fluorkohlenstoffe, MoS₂, WS₂, Talk, Siliconöl, (Poly)silazane, (Poly) siloxane, PTFE und ePTFE. Als besonders vorteilhafte Beschichtungen hat sich zudem eine Kombination aus einem Polysaccharid und einem Protein, insbesondere eine Kombination von Alginat mit Molkeprotein, Sojaprotein usw. sowie mit Mischungen derartiger Proteine erwiesen, deren Gleiteigenschaften und Biokompatibiltät hervorragend ist. Es hat sich zudem als vorteilhaft erwiesen, eine Beschichtung aus Gelatine, Agar-Agar und/oder Cellulose aufzubauen, zu der optional noch weitere Schmierstoffe, insbesondere Triglyceride, zugegeben sein können.

Die Beschichtungsmaterialien liegen zum Beispiel als Nanoteilchen oder Makromoleküle vor. Die Beschichtungsmaterialien können allerdings auch als Mikroteilchen oder Makroteilchen vorliegen.

Durch Einsatz der Beschichtungsmaterialien in Form von Teilchen wie etwa Nanoteilchen lässt sich die Oberflächenstruktur gezielt einregeln, beispielsweise durch schrittweisen Schichtaufbau (layer-by-layer). Die Teilchen können dabei auf der behandelten Oberfläche insbesondere in der Form selbstorganisierter Monoschichten, als Teilchen mit Mikrotextur oder als Nanoteilchen vorliegen, die mit oberflächenaktiven Mitteln bedeckt sind.

Einige weitere der in der Erfindung bevorzugten Methoden zur Oberflächenbehandlung, also zur reinen Beschichtung bzw. Oberflächenmodifizierung oder zur Glättung nach erfolgter Gravur (nachfolgend beschrieben), sollen nachstehend näher beschrieben werden.

Bei einer Keramikbeschichtung wird eine dünne Keramikschicht (Keramikfilm) bei niedriger Temperatur mit einem herkömmlichen Beschichtungsverfahren für Keramikbeschichtung auf die Oberfläche, auf der optional Kavitäten ausgebildet sind, aufgebracht. Durch das Abscheiden eines Keramikfilms lässt sich die behandelte Oberfläche glätten, zudem wird die Biokompatibilität erhöht.

Grundsätzlich lassen sich anorganische Keramik- oder Glasbeschichtungen auf die Oberfläche des Elements durch die in der Technik bekannten Verfahren aufbringen. Als Ausgangssubstanzen, welche für medizintechnische Anwendungen besonders geeignete Beschichtungen ergeben, sind Silane, halogenierte Silane, insbesondere fluorierte Silane, und Silicate auf Basis von Wasserglas sowie Netzwerkstrukturen aus SiO₂, Al₂O₃ und/oder TiO₂, die über das Sol-Gel-Verfahren zugänglich sind.

Eine Silicatisierung einer Oberfläche (einschließlich einer gravierten Oberfläche) erfolgt beispielsweise so, dass eine siliciumorganische Verbindung (Silan) in eine Flamme eingespeist wird. Durch so genannte Flammenpyrolyse wird dann eine dünne, jedoch sehr dichte, gegenüber Feuchtigkeit stabile und festhaftende Silicatschicht mit oftmals hoher Oberflächenenergie auf der bearbeiteten Oberfläche erzeugt. Durch die geringe Einwirkzeit der Flamme kann die Oberflächentemperatur des Werkstücks sehr niedrig gehalten werden. Dadurch ist dieses Verfahren sogar für sehr dünnwandige Kunststoffe geeignet.

Die Silicatschicht haftet praktisch auf allen Oberflächen und bildet eine nanoporöse Oberflächenstruktur, die für eine optimale chemische Anbindung organischer Komponenten, insbesondere z.B. eines Schmiermittels, an die Silicatschicht sorgt.

Alternativ ist es bei Verwendung eines Siliconkautschuks als Material für das der inneren Reibung ausgesetzte Element möglich, durch ein Plasmaverfahren die oberste Siliconschicht thermisch in eine Silicatschicht umzuwandeln. Auf diese Weise wird die oberste Schicht glasartig verhärtet, so dass sich die Vorteile einer verbesserten Härte und einer deutlich glätteren Oberfläche ergeben.

Wird ein Siliconkautschuk als das Material für das der inneren Reibung ausgesetzte Element verwendet, ergeben sich noch weitere Möglichkeiten einer vorteilhaften, die Reibung vermindernden Beschichtung der profilierten oder unprofilierten Oberfläche. So weist der Siliconkautschuk an seiner Oberfläche OH-Gruppen auf, die chemisch so umgewandelt werden können, dass dem Kautschuk hydrophile oder hydrophobe Eigenschaften verliehen werden.

Es ist auch möglich, dass die genannten, für die Beschichtung verwendeten Materialien, insbesondere Graphit, in dem gesamten, aus Siliconkautschuk gebildeten Element vermengt sind. Hierbei kann es zweckmäßig sein, dass geeignete Vermittler zugesetzt sind, welche die Kompatibilität zwischen dem Silicon einerseits und dem darin vermengten Material andererseits erhöhen. Ein Beispiel für einen solchen Vermittler ist etwa Dimethyldisulfid (DMDS), welches die Dispergierfähigkeit insbesondere von Graphit im Siliconkautschuk erhöht.

Wenn das der inneren Reibung ausgesetzte Element selber aus PTFE oder ePTFE gebildet ist, sind ebenfalls Beschichtungen mit den vorstehenden Materialien vorteilhaft einsetzbar. Zudem ist es auch hier möglich, dass diese Materialien, z.B. Graphit, in dem gesamten aus PTFE oder ePTFE gebildeten Element vermengt sind. In diesem Fall kann es ebenfalls für die Verbesserung des Dispersionszustandes günstig sein, dass zusätzlich Vermittler und andere Zusatzstoffe zugegeben sind, beispielsweise geeignete Emulgatoren.

Die wie vorstehend erzeugten Beschichtungen mit (bzw. das Hinzufügen von) z.B. Graphit, Fluorkohlenstoff, MoS₂, WS₂, Talk, Silazanen, Siloxan, PTFE, ePTFE und Siliconöl bzw. Alginat+Protein, insbesondere Alginat+Molkeprotein und/oder Alginat+Sojaprotein, verringern die Reibung weitergehend und vermindern somit den Abrieb bei einem langen Zeitraum der Benutzung.

In dem Fall, dass ein weitgehender Abrieb der Beschichtung, beispielsweise von PTFE, im Laufe der Benutzung auftritt, bildet die Beschichtung eine so genannte Opferschicht. Das erfindungsgemäße Element ist dann vorzugsweise so ausgestaltet, dass selbst nach teilweisem oder völligem Verbrauch der Opferschicht eine Notlaufeigenschaft verbleibt, d.h. eine verminderte, aber die Benutzung immer noch ermöglichende selbstschmierende Eigenschaft.

Eine Modifizierung der Oberfläche durch UV-Bestrahlung ist ebenfalls möglich, wobei die UV-Bestrahlung vorzugsweise im Vakuum vorgenommen wird. Hierbei wird die zu behandelnde Oberfläche bei festgelegtem Druck in einer definierten Atmosphäre, z.B. einer Stickstoff- oder Stickstoff/Sauerstoffatmosphäre, mit UV-Licht, d.h. Licht mit einer Wellenlänge von etwa ≤400 nm, über einen festgelegten Zeitraum bestrahlt. Typische Bestrahlungszeiten, die eine ausreichende Glättung erzielen können, liegen bei 0,5 bis 20 min, insbesondere 1 bis 2,5 min, wobei ab etwa 20 min Bestrahlung meist keine weitere Verbesserung auftritt, so dass solche langen Bestrahlungszeiten wirtschaftlich nachteilig sind. Erfindungsgemäß wird daher bevorzugt für eine Zeit von 0,5 bis 20 min bestrahlt, wobei kürzere Bestrahlungszeiten von z.B. 1 bis 2,5 min vorteilhaft sind, um eine besonders gute Benetzbarkeit zu erzielen. Typische Strahlungsdosen liegen im Bereich von 2 bis 40 J/cm². Die UV-Bestrahlung im Vakuum (VUV-Verfahren) ist besonders vorteilhaft einsetzbar, wenn das der Reibung ausgesetzte Element wenigstens an seiner Oberfläche Siliconkautschuk umfasst und vorzugsweise insgesamt aus Siliconkautschuk gebildet ist. Der Grund liegt darin, dass in diesem Fall eine Nachvernetzung der Siliconoberfläche auftritt, die diese glättet und Klebeeffekte vermeidet bzw. deutlich verringert.

Die Vorteile der UV-Bestrahlung liegen insbesondere im geringen apparativen Aufwand sowie darin, dass weder Verträglichkeitsprobleme noch Haftfestigkeitsprobleme auftreten.

Bei einer Acrylatbeschichtung wird die zu behandelnde Oberfläche mit einer Acrylatschicht ähnlich wie bei einem Farbauftrag beschichtet. Hierzu können vorteilhaft Tauchbeschichtung, Walzenüberziehen, Rakelüberziehen, Rotationsbeschichten (Spin Coating), das so genannte Doctor-Blade-Verfahren usw. eingesetzt werden, um eine gleichmäßige und damit besonders glatte Beschichtung zu erzielen. Gegebenenfalls kann nach der Beschichtung eine Härtung vorgenommen werden. Dabei ist es besonders zweckmäßig, z.B. ein Aushärten durch UV-Bestrahlung durchzuführen. Die erzeugten Acrylatbeschichtungen weisen zweckmäßigerweise eine Dicke von 2 bis 15 µm auf, wobei dünner Beschichtungsdicken von 2 bis 10 µm, insbesondere 2 bis 5 µm unter dem Gesichtspunkt der Biegsamkeit bevorzugt sind.

Als Acrylat kann jedes in der Technik für diesen Zweck bekannte Acrylat eingesetzt werden. Die geeignete Auswahl des Acrylats erlaubt es, die Eigenschaften der Oberfläche des Elements, das einer inneren Reibung ausgesetzt ist, einzuregeln.

Es ist des Weiteren möglich, eine Glättung der profilierten Oberfläche vorzunehmen, indem eine Polysilazanschicht erzeugt wird, entweder eine organische oder eine anorganische Polysilazanschicht. Solch eine Polysilazanschicht kann vorteilhaft durch Tauchbeschichtung und anschließende oxidative Umwandlung aufgebracht werden. Hierbei wird z.B. Perhydropolysilazan durch Eintauchen aufgebracht und oxidiert. Auf diesem Weg können auch sehr dünne Beschichtungen ausgebildet werden, die für die Biegsamkeit des beschichteten Elements vorteilhaft sind, beispielsweise in einer Dicke von 0,01 bis ≤1 µm.

Es hat sich zudem gezeigt, dass auf diese Weise sehr gute Haftfestigkeiten erzielt werden können, wobei weniger problematische, also etwa keine bzw. kaum reizende oder ätzende Substanzen eingesetzt werden, was in medizintechnischen Anwendungen die Genehmigungsfähigkeit im Einzelfall stark verbessern kann. Zudem ist die Produktpalette der Polysilazane sehr groß, so dass genau auf die Anwendung und erwünschte Eigenschaften abgestimmte Rohmaterialien ausgewählt werden können.

Die vorstehend erwähnte Arylbeschichtung erfolgt vorteilhaft durch das CVD-Verfahren, wobei Arylverbindungen als Precursor eingesetzt werden. Beispiele für Precursorverbindungen sind substituierte Benzole wie etwa o-, m- oder p-Xylol oder auch das Di-p-Xyloldimer.

Als für medizintechnische Anwendungen besonders gut geeignete organische Beschichtungen sind zudem 2-Komponenten-Epoxid-Einbrennbeschichtungen, Polyurethan-Einbrennbeschichtungen, 2-Komponenten-Polyurethan lufttrocknende Lacke sowie fluorierte Polymere zu erwähnen. Für die fluorierten Polymere sind Polytetrafluorethylen, fluoriertes Ethylen/Propylen-Copolymer, Perfluoralkoxycopolymer, Polyvinylidenfluorid und Ethylenchlortrifluorethylen besonders gut geeignet.

Um gezielt eine hydrophile Oberfläche einzustellen, ist zudem eine Polyacrylnitrilbeschichtung vorteilhaft, während mittels einer Polysulfonbeschichtung gezielt eine hydrophobe Oberfläche eingestellt werden kann.

Für die vorstehend aufgeführten Beschichtungsmethoden ist es weitergehend vorteilhaft, dass diesen eine Vorbehandlung vorausgeht. Besonders gute Haftfestigkeit und Gleitverhalten sind erzielbar, wenn insbesondere eine Corona-Aktivierung, eine Fluorierung oder ein Beflammen als Vorbehandlung eingesetzt wird, von denen die Corona-Aktivierung die besten Ergebnisse liefert.

Auch durch die gezielte Beschichtung des Grundelements mit einer weiteren Substanz, d.h. durch den Aufbau einer oder mehrerer Schichten auf einer ggf. vorbehandelten Oberfläche, lässt sich die Oberflächenenergie der resultieren Oberfläche einstellen, welche letztendlich der Reibung ausgesetzt ist.

Das selbstschmierende Element kann auch aus einem nicht selbstschmierenden Grundelement sowie einem zu diesem hinzugefügten Schmiermittel bestehen. Auch kann zur weiteren Verminderung des Reibungswiderstands zu einem der vorstehend erläuterten selbstschmierenden Elemente ein weiteres Schmiermittel hinzugefügt sein.

Um eine besonders gute Absonderung des Schmiermittels sowie eine Bevorratung des Schmiermittels direkt an der Oberfläche des selbstschmierenden Elements zu gewährleisten, ist es bevorzugt, dass wenigstens eine Oberfläche des selbstschmierenden Elements ein Profil aufweist, dessen Kavitäten ein Schmiermittel beinhalten. Es ist insbesondere bevorzugt, dass derartige Kavitäten zusätzlich in einem selbstschmierenden Element ausgebildet sind, welches nach Erzeugung der Kavitäten der vorstehend beschriebene Plasmabehandlung unterzogen wurde, vorzugsweise ein aus Silicon, (e)PTFE oder Polyurethan bestehendes selbstschmierendes Element, welches mit einer Siloxan- oder Perfluorcarbonbeschichtung versehen ist, die durch Plasmabehandlung ausgebildet ist, wobei die Kavitäten mit der Beschichtung überzogen sind.

Ein derartiges profiliertes (d.h. mit Schmiermittel enthaltenden Kavitäten versehenes) selbstschmierendes Element kann insbesondere vorteilhaft als Stülpschlauch und/oder Schafthülle für ein Endoskop eingesetzt werden, insbesondere bei einer Untersuchung des Dünndarms. Dabei bilden die Kavitäten quasi ein Reservoir für zusätzlichen Schmierstoff aus, der dann in den engen Kurven eine bessere, d.h. reduziertere Reibung durch eine Restschmierung erzielt.

Dies kann unter anderem auch durch ein bei der Herstellung appliziertes Fett erreicht werden, welches in den Kavitäten sowie optional zusätzlich außerhalb von diesen vorliegt und das bei Körpertemperatur flüssiger wird als außerhalb des Körpers. Im Gegensatz zu einer vor und/oder während der Untersuchung vorgenommenen Schmierung des Schaft- und/oder Stülpschlauchsystems des Endoskops ist die einmalige Befüllung der Kavitäten wesentlich einfacher als eine während der Untersuchung notwendige Schmierung.

Diese Kavitäten, welche das Profil ausbilden, können regelmäßig oder unregelmäßig ausgebildet sein. Die Tiefe der ausgebildeten Kavitäten beträgt etwa 10 bis 250 µm, bevorzugt 50 bis 200 µm und insbesondere bevorzugt 100 bis 150 µm. Bei einer zu geringen Tiefe kann die aufgenommene Schmiermittelmenge unzureichend sein, bei einer zu großen Tiefe werden bei der Erzeugung der Kavitäten leicht unvorteilhafte Schädigungen der Kavitätenränder erzeugt. Um einen guten Ausgleich zu schaffen, liegt die Tiefe vorzugsweise im genannten Bereich. Aus dem gleichen Grund weisen die Kavitäten bevorzugt einen Durchmesser von 50 bis 250 µm, mehr bevorzugt 100 bis 200 µm, weiter bevorzugt 130 bis 170 µm und insbesondere von ca. 150 µm auf.

Grundsätzlich können alle hier beschriebenen Schmiermittel eingesetzt werden, wobei Schmiermittel auf Pflanzenbasis bevorzugt sind, insbesondere Palmfett, da dieses gut verträglich ist, ferner leicht zu handhaben ist und in einfacher Weise in das Profil eingefüllt werden kann.

Wenn Schmiermittel enthaltende Kavitäten an wenigstens einer Oberfläche des'Elements vorliegen, ist es möglich, dass die Kavitäten durch äußere Einflüsse ganz gezielt und zu einem vorbestimmten Zeitpunkt Schmiermittel freisetzen. Die Kavitäten, z.B. in der Form von Nuten, können vorteilhaft durch Prägen, Aufschäumen und Aufschneiden, Schneiden oder Walzen oder durch eine Gravur, insbesondere eine Lasergravur, erzeugbar sein.

In einer bevorzugten Ausführungsform der Erfindung befindet sich das Schmiermittel daher in Nuten, die direkt an einer Oberfläche des Elements angeordnet sind. In einigen Ausgestaltungen bilden regelmäßig angeordnete Nuten, in anderen Ausgestaltungen unregelmäßig angeordnete Nuten das Profil. Bevorzugte Ausführungen des Profils sind nachstehend detaillierter beschrieben.

Das vorstehend erwähnte Profil ist vorzugsweise so angepasst, dass freigesetztes Schmiermittel zu den einer besonders großen Reibung ausgesetzten Bereichen zugeführt wird. Hierzu kann das Profil längsverlaufende, querverlaufende oder spiralförmig verlaufende Kavitäten aufweisen, die vorzugsweise durch Nuten bzw. Rillen oder durch einzelne, lokal begrenzte Vertiefungen gebildet werden.

Oftmals kommen in unterschiedlichen Anwendungen auch zwei getrennte bzw. unterschiedliche Abschnitte des selbstschmierenden Elements miteinander in Kontakt, was zu erhöhter Reibung an dieser Stelle führt. Daher ist es besonders vorteilhaft, wenn das Profil so angepasst ist, dass bei Überlagerung zweier entgegenstehender Abschnitte des selbstschmierenden Elements die jeweiligen Profilabschnitte nicht ineinander greifen können.

Im Falle eines tubusartigen bzw. schlauchförmigen Elements sind daher vorzugsweise Nuten vorgesehen, die mit der Mantellinie des schlauchförmigen Elements einen Winkel von größer als 0° bis kleiner als 90° einschließen.

Es hat sich für die vorliegende Erfindung als besonders geeignet erwiesen, dass das Profil eine bevorzugte Tiefe von 50 bis 500 µm und mehr bevorzugt 200 bis 400 µm aufweist. Bei einer Tiefe von weniger als 50 µm kann nur begrenzt Schmiermittel aufgenommen werden, die selbstschmierende Wirkung lässt relativ schnell nach. Andererseits führen Tiefen von größer als 500 µm leicht zu schlechteren Abmessungsgenauigkeiten bei der Herstellung. Zudem erfährt die behandelte Oberfläche bei der Erzeugung solch eines Profils von tiefer als 500 µm stärkere

Einwirkungen (z.B. mechanische oder thermische), so dass leicht größere Unebenheiten auf der Oberfläche erzeugt werden, was die Gleitwirkung mindert. Daher ist der Bereich von 50 bis 500 µm in der vorliegenden Erfindung bevorzugt, wobei die beschriebenen Nachteile im Bereich von 200 bis 400 µm noch weiter vermindert sind.

Ein zum Zweck der Schmiermittelaufnahme und kontrollierten Schmiermittelabgabe besonders geeignetes Profil lässt sich durch Gravieren, insbesondere durch Lasergravieren (auch als Laserengraving bekannt) erzeugen. Es ist daher bevorzugt, dass das Profil durch Gravieren mit einem Laser erzeugbar ist. Ein selbstschmierendes Element, das ein durch Lasergravieren erzeugbares Profil aufweist, ist daher ebenfalls in die vorliegende Erfindung eingeschlossen.

Das Lasergravieren erlaubt einen kontrollierten Materialabtrag, der z.B. mittels eines gepulsten Laserstrahls stattfindet. Der Strahl wird dabei vorzugsweise zur Verbesserung der Bearbeitungsgenauigkeit sehr stark fokussiert, wobei z.B. Brennpunkte mit einem Durchmesser 0,01 bis 0,1 mm erzeugt werden. Im Brennpunkt können sehr hohe Temperaturen von beispielsweise größer 2000°C erreicht werden, was eine schnelle Bearbeitung nahezu beliebiger Materialien ermöglicht.

Da der Materialabtrag auf Grundlage von thermischen Prozessen stattfindet, spielt die Härte des zu bearbeitenden Werkstoffes eine untergeordnete Rolle. Das Verfahren ist daher für alle erfindungsgemäß verwendbaren Werkstoffe einsetzbar. Außerdem ergibt sich gegenüber anderen einsetzbaren Verfahren, wie z.B. dem Erodieren, eine Zeitersparnis von bis zu 60%.

Beim Lasergravieren wird das abzutragende Material nur lokal geschmolzen und verdampft, was ein sehr präzises Arbeiten erlaubt. Dabei hängt die Präzision unter anderem vom Brennpunktdurchmesser des fokussierten Strahls und von der Genauigkeit des Lasers ab. Beide Parameter werden daher erfindungsgemäß bevorzugt auf Werte von ≤0,1 mm eingestellt.

Für den Materialabtrag spielt die Wellenlänge des Lasers ebenfalls eine wichtige Rolle, d.h. in welchem Maß die eingestrahlte Energie absorbiert wird. Der Absorptionsgrad wiederum hängt vom Werkstoff und von der Wellenlange des verwendeten Lasers ab. Die Auswahl geeigneter Wellenlängen ist daher sehr wichtig und wird in der vorliegenden Erfindung vorzugsweise so ausgewählt, dass der bearbeitete Werkstoff die Laserenergie zu mindestens 50% oder mehr, mehr bevorzugt zu mindestens 60% oder mehr und insbesondere bevorzugt zu mindestens 70% oder mehr absorbiert.

Erfindungsgemäß kommen beim Lasergravieren z.B. Festkörperlaser zum Einsatz. Diese Laser können lampen- oder diodengepumpt arbeiten. Ihre Ursprungswellenlängen befinden sich gewöhnlich im IR-Bereich, können aber mittels spezifischer optischer Kristalle bis in den Ultraviolett-Bereich (UV) gebracht werden. Dies ist sowohl wegen der Absorptionseigenschaften verschiedener Materialien als auch wegen der Fokussierbarkeit vorteilhaft. UV-Laserstrahlen können im Vergleich zu IR-Laserstrahlen auch auf kleinere Brennpunktdurchmesser fokussiert werden.

Daneben ist für eine besonders filigrane und leicht zu steuernde Bearbeitung die Verwendung eines Excimer-Lasers oder eines CO₂-Lasers besonders bevorzugt. Das Lasern mit einem CO₂-Laser ist billiger und daher vorteilhafter. Der CO₂-Laser arbeitet vorzugsweise mit einer Wellenlänge von 10600 nm. Die Pulsenergie ist vorteilhafter Weise zwischen ca. 10 und 100 µs und insbesondere zwischen 20 und 60 µs eingestellt.

Bei einem Gravierungsschritt können, seitlich von den erzeugten Kavitäten, Aufwerfungen auf der behandelten Oberfläche auftreten. Derartige Aufwerfungen verringern die selbstschmierenden Eigenschaften des Elements. Daher ist es weitergehend bevorzugt, dass das Profil durch Gravieren mit einem Laser und ein anschließendes Glätten erhältlich ist.

Als besonders geeignete Methoden zum Glätten der gravierten Oberfläche, so dass die Aufwerfungen nivelliert werden und gleichzeitig eine zur Aufnahme einer ausreichenden Schmiermittelmenge geeignete Tiefe der eingravierten Kavitäten verbleibt, haben sich die bereits vorstehend aufgeführten Verfahren erwiesen. So können insbesondere Keramikbeschichtung, Silicatisierung, UV-Bestrahlung, Acrylatbeschichtung, Arylbeschichtung, Polysilazanbeschichtung und Polysiloxanbeschichtung dazu eingesetzt werden, die erforderliche Glättung schnell und einheitlich zu erzielen. Bei geeigneten Behandlungszeiten und -bedingungen kann auch eine Plasmabehandlung geeignet sein, eine Glättung hervorzurufen, insbesondere wenn es sich um die Plasmapolymerisation oder ein Aufpfropfen von Polymer(en) nach einer Plasmabehandlung handelt.

In einer weiteren bevorzugten Ausführungsform der Erfindung weist das selbstschmierende Element eine Schichtstruktur auf. Die Schichtstruktur umfasst mindestens eine Substratschicht, um eine stabile Grundlage für darauf angeordnete Schichten auszubilden, eine auf mindestens einer, vorzugsweise beiden Seiten des Substrats angeordnete Reservoirschicht für Schmiermittel, um genügend Schmiermittel für den tatsächlichen Einsatz zu bevorraten, und mindestens eine bzw., wenn auf beiden Seiten des Substrats eine Reservoirschicht angeordnet ist, zwei außenliegende perforierte Schicht(en), um das Schmiermittel in kontrollierter Weise abzugeben. Im ersten Fall ergibt sich der grundsätzliche Aufbau Substrat/Reservoirschicht/perforierte Schicht, im zweiten Fall der grundsätzliche Aufbau perforierte Schicht/Reservoirschicht/Substrat/Reservoirschicht/perforierte Schicht, wobei jeweils noch Zwischenschichten vorgesehen sein können.

Die Substratschicht kann z.B. aus einem der bereits vorstehend aufgeführten Kautschuke gebildet sein, wobei Siliconkautschuk aufgrund der angegebenen Vorteile besonders bevorzugt ist. Die Substratschicht ist so ausgestaltet, dass sie für das verwendete Schmiermittel im. Wesentlichen undurchlässig ist.

Die Reservoirschicht kann so ausgebildet sein, dass das Schmiermittel in fester Form als Schmiermittelschicht ausgebildet ist. Das Schmiermittel ist dann so angepasst, dass es durch äußere Einflüsse, wie sie später beschrieben werden, erweicht oder sich verflüssigt und an mindestens eine benachbarte Schicht abgegeben wird.

Zudem ist es möglich, dass die Reservoirschicht mit einer schwammartigen Struktur ausgebildet oder durch ein Material, welches Kapillarwirkung entfaltet, gebildet ist, wobei das Schmiermittel von der schlammartigen Struktur bzw. durch das Material aufgesogen ist und über die Zeit bzw. wiederum bei Auftreten spezieller äußerer Einflüsse an mindestens eine benachbarte Schicht abgegeben wird.

Im dem Fall, dass zwei Reservoirschichten vorliegen, können diese gleich oder unterschiedlich ausgebildet sein. Eine unterschiedliche Ausgestaltung kann insbesondere vorteilhaft sein, wenn unterschiedliche Druckeinflüsse vorliegen. Wenn z.B. das erfindungsgemäße selbstschmierende Element als ein Stülpschlauch ausgebildet ist, treten insbesondere im Umstülpbereich unterschiedliche physikalische Einflüsse wie z.B. Druck bei der innenliegenden und der außenliegenden Reservoirschicht auf, so dass hier eine unterschiedliche Ausgestaltung dieser Schicht, die jeweils den äußeren Bedingungen angepasst ist, nützlich sein kann. Dieselbe Überlegung gilt auch für die hier nachstehend beschriebene perforierte Schicht.

Vorzugsweise ist die perforierte Schicht direkt angrenzend an die Reservoirschicht bereitgestellt und bildet die äußere Oberfläche des Elements aus. Allerdings können zwischen der Reservoirschicht und der perforierten Schicht weitere Schichten angeordnet sein, solange sie für das Schmiermittel im Wesentlichen durchlässig sind. Die Perforation der perforierten Schicht kann jede beliebige Gestalt aufweisen. Es ist bevorzugt, dass diese Schicht durchgehende Löcher bzw. Kanäle aufweist, deren Mittelachsen mit der Oberfläche des Elements, dessen Oberfläche durch die perforierte Schicht gebildet wird, einen nahezu rechten Winkel einschließen, also von z.B. 80 bis 100°, insbesondere von etwa 90°. Auf diese Weise gelangt das Schmiermittel in besonders effektiver und kontrollierter Weise von der Reservoirschicht an die zu schmierende Oberfläche.

Zudem ist es möglich, die perforierte bzw. poröse Schicht durch eines der vorstehend beschriebenen Plasmaverfahren auszubilden.

Das schichtweise aufgebaute Element erlaubt eine einfache Herstellung, indem z.B. die einzelnen Schichten wie erforderlich vorausgehend hergestellt und dann' zusammenlaminiert werden oder indem die Schmiermittelschicht auf das Substrat bzw. die oberste von ggf. über dem Substrat angeordneten Schichten aufgebracht und dieser Verbund dann mit einer perforierten Schicht versehen wird. Ein Vorteil dieser Ausführungsform ist, dass das Schmiermittelreservoir mit beliebigen, anwendungsabhängigen Größen ausgestaltet werden kann.

Bei der inneren Reibung der medizintechnischen Vorrichtung wird Schmiermittel z.B. durch physikalische oder chemische Einflüsse freigesetzt. Zu den physikalischen Einflüssen zählen hier beispielsweise Druck- und Temperatureinflüsse oder -Veränderungen. Insbesondere treten Druckeinflüsse auf, wenn sich die-medizintechnische Vorrichtung bei ihrer Verwendung körperlichen Gegebenheiten anpasst. Typischer Weise tritt eine solche Situation in der Endoskopie auf, wenn das distale Ende, d.h. dass mit dem Körper in Eingriff gebrachte bzw. eingeführte Ende, beim Vortrieb abgewinkelt wird.

Als Beispiele für chemische Einflüsse können saure (pH = 0 bis 6,99) und basische (pH = 7,01 bis 14) Lösungen angeführt werden, die z.B. durch Eintauchen in die Lösungen auf das Gegenelement aufgebracht werden können. Die sauren oder basischen Lösungen führen zu einer chemischen Veränderung des Schmiermittels, die Einfluss auf dessen Erweichungs- oder Schmelzpunkt hat, also diesen etwa herabsetzt, so dass Schmiermittel austritt.

Damit möglichst keine bzw. keine übermäßige Reaktion zwischen saurer bzw. basischer Lösung und dem Werkstoff auftritt, sollten deren pH-Werte bevorzugt bei 5 bis 6,99 (saure Lösung) bzw. 7,01 bis 9 (basische Lösung) liegen. Unter physiologischen Gesichtspunkten ist in medizintechnischen Anwendungen ein pH von 5 bis 6,99 am meisten bevorzugt.

Der chemische Einfluss muss darüber hinaus nicht notwendigerweise durch saure oder basische Lösungen ausgeübt werden. Für diese Ausführungsform reicht es aus, dass durch chemische Wechselwirkung eine Freisetzung von Schmiermittel erfolgt.

Ferner ist es bevorzugt, dass das einer inneren Reibung ausgesetzte Element aus einem Werkstoff gebildet ist, der einen Kautschuk und ein Schmiermittel umfasst. Die Verwendung eines Kautschuks bzw. Kautschukgemisches erlaubt eine besonders flexible Ausgestaltung des Elements, was insbesondere im Hinblick auf die medizintechnische Verwendung, vorzugsweise im Bereich der Endoskopie, vorteilhaft ist.

Aufgrund seiner einfachen Verarbeitbarkeit und seiner Wirtschaftlichkeit wird bevorzugt ein Siliconkautschuk für den Kautschuk verwendet. Zudem kann durch Gravieren mit einem Laser, sofern dieses zum Einsatz kommt, besonders leicht eine hohe Abmessungsgenauigkeit erzielt werden. Dies wirkt sich vorteilhaft auf die Gleiteigenschaften aus. Geeigneter Weise ist das Schmiermittel Siliconöl, da dieses chemisch inert ist und seine Eigenschaften anwendungsbezogen leicht eingeregelt werden können. Aufgrund der hohen Kompatibilität zwischen Siliconkautschuk einerseits und Siliconöl andererseits stellt diese Kombination eine besonders bevorzugte Kombination von Kautschuk und Schmiermittel dar. Im Falle der Profilerzeugung durch Gravieren kommt vorzugsweise pflanzliches Schmiermittel, z.B. Palmfett, zum Einsatz.

Wie vorstehend erwähnt umfasst das selbstschmierende Element der medizintechnischen Vorrichtung bevorzugt einen selbstschmierenden Kautschuk und ein Schmiermittel. Die Kautschuke können zu beliebigen Gestalten ausgeformt sein. Sie sind in der Regel vergleichsweise preiswert, was die Massenproduktion, insbesondere von Einwegartikeln, ermöglicht. Ferner können die Eigenschaften der Kautschuke, wie beispielsweise die Aufnahmekapazität für Schmiermittel, Flexibilität, Formstabilität bei erhöhten Temperaturen usw., sehr leicht eingestellt werden.

Als Schmiermittel kommen insbesondere ölartige und gelartige Schmiermittel sowie Öl-in-Wasser-Emulsionen (O/W-Emulsionen), Wasser-in-Öl-Emulsionen (W/O-Emulsionen) und Stickstoffemulsionen in Betracht.

Grundsätzlich sind alle Arten von Ölen geeignet, wie etwa pflanzliche Öle, tierische Öle, mineralische Öle und synthetische Öle.

Besonders gut geeignete Schmiermittel sind Paraffinöl, insbesondere dickflüssiges Paraffin (lat. Bez. paraffinum subliquidum) und Glycerin, weil diese in der Regel von Menschen und vielen Tieren gut vertragen werden. In einer bevorzugten Ausführungsform wird aufgrund seiner Erhältlichkeit, seiner guten Schmiereigenschaften und guten Verarbeitbarkeit sowie der hervorragenden Biokompatibilität Palmfett eingesetzt. Insbesondere in dem Fall, dass ein Profil erzeugt und mit Schmiermittel befüllt wird, ist Palmfett vorteilhaft.

Hervorragend geeignet sind auch Siliconöle, weil diese insbesondere in Kautschuken auf Siliconbasis, wie sie vorstehend beschrieben wurden, gut gespeichert und von diesen kontrolliert abgegeben werden können, so dass eine kontinuierliche Schmierwirkung sichergestellt werden kann. Besonders vorteilhafte Gleiteigenschaften werden erzielt, indem ein mit Graphit vermengtes Siliconöl eingesetzt wird. Im Falle einer vorhergehend beschriebenen Beschichtung des Siliconkautschukelements mit PTFE wird bevorzugt eine Silicon/Wasser-Emulsion eingesetzt, da hierdurch die Wechselwirkungen zwischen Schmiermittel und Beschichtung verbessert werden können.

Die medizintechnische Vorrichtung kann ein Schmiermittel auf unterschiedlichste Weise umfassen. In einer bevorzugten Ausführungsform der vorliegenden Erfindung ist das Schmiermittel nahezu homogen in dem Werkstoff verteilt, der das selb.stschmierende Element bildet. Dies ist zum Beispiel dadurch möglich, dass das Schmiermittel bei der Herstellung des Werkstoffs, insbesondere eines Kautschuks, zugegeben und mit diesem oder dessen Ausgangssubstanzen vermengt wird. Ein typisches Beispiel für solch einen Werkstoff ist ein Siliconkautschuk, der des Weiteren ein Siliconöl enthält.

Gewöhnlich besteht in dem vorstehend beschriebenen Fall, dass das Schmiermittel nahezu homogen im Werkstoff verteilt ist, eine gewisse Unverträglichkeit zwischen Schmiermittel einerseits und dem Werkstoff andererseits. Diese Unverträglichkeit beruht auf einem Unterschied im chemischen Potential zwischen dem Schmiermittel und dem Werkstoff. Dadurch kommt es zu einer fortschreitenden Trennung, vergleichbar einer Trennung zwischen einer Öl- und einer Wasserphase. Die Trennung bewirkt, dass das Schmiermittel "ausblutet" oder "ausschwitzt", so dass auf einer Oberfläche des selbstschmierenden Elements der medizintechnischen Vorrichtung ein Schmiermittelfilm ausgebildet wird.

Die Vorteile dieser Ausführungsform liegen in der einfachen Herstellbarkeit, der großen Breite von möglichen Ausgangs- und Inhaltsstoffen sowie der kontinuierlichen Schmierung über einen langen Zeitraum.

Die vorstehend beschriebene Trennung von Schmiermittel und Werkstoff führt zu einer zunehmenden Inhomogenität des Schmiermittels im Werkstoff.

Wenn ein kurzfristiges starkes Ausbluten (Ausschwitzen) des Schmiermittels erwünscht ist, kann es zudem vorteilhaft sein, wenn das Element, das der inneren Reibung ausgesetzt ist, porös ist. In dem Element enthaltenes Schmiermittel kann dann beispielsweise bei einer äußeren Druckerhöhung schneller an die Oberfläche des Elements gelangen und dort die Schmierleistung signifikant steigern.

In ähnlicher Weise ist es für ein vergleichsweise kurzes, aber intensives Ausbluten von Schmiermittel von Vorteil, wenn die Viskosität des Schmiermittels bei der tatsächlichen Benutzungstemperatur absinkt. Wenngleich ein solches Absinken nicht speziell beschränkt ist, sind Viskositätsverringerungen um mindestens 2,5%, bevorzugt 5%, mehr bevorzugt 7,5% bis hin zu 10%, 20% oder sogar 50% oder darüber besonders vorteilhaft. Als Referenz kann hier beispielsweise die so genannte Mooney-Viskosität des Schmiermittels dienen, z.B. ML₁₊₄(100°C).

Für das Schmiermittel ist es bevorzugt, dass seine Temperatur-Viskositäts-Charakteristik im Temperaturbereich von 10-43°C eine Unstetigkeit aufweist. Bei der medizintechnischen Verwendung, beispielsweise in der Endoskopie, kann das Element z.B. in einem handelsüblichen Kühlschrank gelagert werden und entfaltet dann bei der tatsächlichen Verwendung, etwa bei Raumtemperatur oder bei in menschlichen oder tierischen Körpern vorherrschenden Temperaturen, aufgrund der vergrößerten Fluidität des Schmiermittels eine erhöhte Schmierwirkung.

Es ist daher bevorzugt, wenn sich bei Raumtemperatur, beispielsweise zwischen 10 bis 30°C, bevorzugt zwischen 20 und 25°C, eine deutliche Viskositätsverringerung zeigt. Anders ausgedrückt ist es bevorzugt, dass die Temperatur-Viskositäts-Charakteristik des Schmiermittels im Temperaturbereich von 10-30°C, bevorzugt zwischen 20 und 25°C, eine Unstetigkeit aufweist, beispielsweise einen Sprung, insbesondere einen Sprung, der eine der vorstehend angegebenen prozentualen Veränderungen ausmacht.

Gleichfalls ist es sehr vorteilhaft, wenn das vorstehend beschriebene Absinken der Viskosität des Schmiermittels bei Temperaturen auftritt, die gewöhnlich in menschlichen oder tierischen Körpern vorherrschen. Für einige Ausführungsformen ist daher ein Viskositätsabfall mit dem vorstehend beschriebenen Ausmaß im Temperaturbereich von 30 bis 43°C, bevorzugt im Bereich von 36 bis 38°C, insbesondere um 37±0,5°C herum, erwünscht. D.h., das Schmiermittel weist in seiner Temperatur-Viskositäts-Charakteristik im genannten Temperaturbereich eine Unstetigkeit auf, wobei wiederum die oben genannten prozentualen Veränderungen besonders bevorzugt sind.

In der vorliegenden Erfindung können bevorzugte Schmelzpunkte für das verwendete Schmiermittel auch im Bereich von 50°C liegen, bei etwa 50°C oder darüber. Auch ist es bevorzugt, dass das Schmiermittel bei Körpertemperatur, etwa 37°C, noch nicht vollständig schmilzt, sondern hier die Verflüssigung erst einsetzt.

Natürlich kann auch in diesen Fällen eines Viskositätsabfalls bei vorbestimmten Temperaturen das Element porös sein, was die genannten vorteilhaften Wirkungen weiter verstärkt.

Oftmals können die verwendeten Werkstoffe für das selbstschmierende Element, insbesondere Kautschuke, durch ein darin enthaltenes Verstärkungsmittel mit einer noch besseren Formstabilität ausgestattet werden. In diesem Fall ist es möglich, dass das Verstärkungsmittel mit einem Schmiermittel getränkt ist, was ebenfalls eine effektive Speicherung des Schmiermittels im Werkstoff erlaubt.

Selbstverständlich kann ein Verstärkungsmittel auch in den vorstehend beschriebenen Ausführungsformen der Speicherung des Schmiermittels vorliegen, wobei es in diesen Ausführungsformen ebenfalls mit dem Schmiermittel getränkt sein kann, aber nicht zu sein braucht.

Bevorzugte Beispiele für Verstärkungsmittel sind Textilverbundstoffe, die gewoben oder nicht-gewoben sein können. Die Textilverbundstoffe können in einer oder mehreren Richtungen nahezu parallel ausgerichtete oder zufällig angeordnete textile Fäden aufweisen. Der Textilverbundstoff kann auch im Wesentlichen teilchen- bzw. pulverförmig vorliegen.

Als Materialien für die Textilverbundstoffe eignen sich Wolle und Baumwolle in zahlreichen Ausgestaltungen besonders gut, insbesondere, wenn eine große Kompatibilität zwischen Schmiermittel einerseits und dem (baum)wollen Textilverbundstoff andererseits vorliegt. In Abhängigkeit vom gewählten Schmiermittel kann es zudem bevorzugt sein, synthetische Textilverbundstoffe zu verwenden, bevorzugt nylonartige, dralonartige und/oder rayonartige synthetische Textilverbundstoffe.

Ein weiteres Beispiel ist, dass auf einer oder mehreren Oberflächen des Elements eine Schmierstofflage ausgebildet ist, die unter Druck- und/oder Wärmeeinwirkung erweicht und eine Schmierwirkung entfaltet. Auf diese Weise kann das selbstschmierende Element aus jedem geeigneten Material bestehen, das in einfacher Weise mit der Schmiermittellage beschichtet werden kann. Diese Schmiermittellage ist erneuerbar, so dass auch wiederholte Verwendungen möglich sind.

Es ist ferner möglich und bevorzugt, dass das selbstschmierende Element schwammartig ausgebildet oder durch ein Material, welches Kapillarwirkung entfaltet, gebildet ist. Das Schmiermittel wird in diesem Fall von dem Element eingesogen und beispielsweise durch Chemisorption oder Physisorption in Hohlräumen, beispielsweise Poren oder Kanälen, gehalten. Ferner kann ein Werkstoff, der das selbstschmierende Element bildet, insbesondere im Falle von Kautschuken gemeinsam mit'darin enthaltenem Schmiermittel hergestellt und eine Hohlraumerzeugung anschließend beispielsweise durch Aufschäumen erzielt werden. Eine solche schwammartige Struktur bzw. solch ein Kapillarwirkung entfaltendes Material ist auch besonders gut für die vorstehend beschriebene Reservoirschicht des ebenfalls vorstehend beschriebenen Laminats geeignet.

Indem z.B. Druck auf ein derart mit Schmiermittel befülltes Element ausgeübt wird, kann das Schmiermittel wieder freigesetzt werden, so dass sich wiederum ein Schmiermittelfilm an einer oder mehreren Oberflächen des Elements ausbildet und somit eine Selbst- bzw. Eigenschmierung erfolgt.

Das selbstschmierende Element der medizintechnischen Vorrichtung der Erfindung kann grundsätzlich verschiedene Formen aufweisen und kann beispielsweise eine rechtwinkelige oder kreisrunde Scheibe, ein rechwinkeliger oder mehrkantiger Tubus, eine Spirale oder ein Schlauch sein. In vielen Anwendungen, z.B. in der Endoskopie, ist es jedoch bevorzugt, dass das Element röhren- bzw. schlauchförmig ist.

Im Falle eines selbstschmierenden Elements in Schläuchform ist es möglich, dass nur die Außenseite des Schlauches selbstschmierende Eigenschaften aufweist, dass nur die Innenseite des Schlauches selbstschmierende Eigenschaften aufweist oder dass sowohl die Außen- als auch die Innenseite des Schlauches selbstschmierende Eigenschaften aufweisen. Dass beide Seiten selbstschmierende Eigenschaften aufweisen, ist insbesondere dann bevorzugt und vorteilhaft, wenn der Schlauch ein so genannter Stülpschläuch ist, der an einer Seite umgestülpt ist und somit auf sich selber zu liegen kommt. Dabei gleitet dann ein Abschnitt der Außenseite auf einem anderen Abschnitt der Außenseite. Ist ein weiteres Element, z.B. ein Endoskopschaft, in den Schlauch eingeführt, wird dieser bei der Relativbewegung des Schlauches vorwärtsgetrieben. Neben der verminderten Reibung zwischen den Abschnitten des Stülpschlauchs selber wird zudem die Reibung mit dem eingeführten Element, etwa dem Endoskopschaft, herabgesetzt, wobei dieser Schaft selber weitergehend ummantelt sein kann, beispielsweise mit einem weiteren Schlauch. Außerdem wird auch die Reibung zwischen dem Stülpschlauch einerseits und seiner Umgebung, i.d.R also den Wänden der Körperöffnung, herabgesetzt.

Sofern ein zusätzlich von außen zugeführtes Schmiermittel (z.B. ein Öl) zum Einsatz kommt, kann dieses zum einen zwischen dem selbstschmierenden Element und dem Schaft bzw. dem Außenraum und anderseits in dem Raum vorliegen, der beim Umstülpen zwischen zwei Schlauchabschnitten ausgebildet wird.

Typische Längen eines derartigen Stülpschlauchs (im abgerollten Zustand), liegen im Bereich von 3 m, üblicherweise 3,20 bis 3,50 m. Bei bestimmten Anwendungen, insbesondere in der Endoskopie des Dünndarms, sind gegebenenfalls allerdings auch über 3,50 m hinausgehende Längen erforderlich. Die hier angegeben Längen beziehen sich insbesondere auf Untersuchungen am Menschen. Bei tiermedizinischen Anwendungen sind daher entsprechend angepasste, also deutlich kürzere aber auch größere Längen, zu berücksichtigen.

Für endoskopische und andere Anwendungen ist es ferner vorteilhaft, wenn das erfindungsgemäße selbstschmierende Element biegsam bzw. flexibel ist. Dabei kann das Element elastische oder inelastische Eigenschaften aufweisen, wobei inelastische Eigenschaften bevorzugt sind, da sie aufgrund der resultierenden Formstabilität eine bessere mechanischen Kontrolle der Vorrichtung ermöglichten.

Die selbstschmierenden Eigenschaften machen sich bei den inneren Reibungsvorgängen vorteilhaft bemerkbar. Diese Reibungsvorgänge können eine Reibung zwischen unterschiedlichen Abschnitten des selbstschmierenden Elements sowie eine Reibung zwischen dem Element und einem weiteren Gegenelement umfassen.

Eine Reibung zwischen zwei unterschiedlichen Abschnitten des Elements tritt bei einem so genannten Stülpschlauch auf, bei dem ein Schlauch so eingerollt ist, dass eine vom Mittelpunkt des Schlauchs aus gesehen außenliegende Oberfläche umstülpt und mit sich selber in Kontakt tritt.

Aufgrund der vorstehend beschriebenen jeweiligen Vorteile kann die medizintechnische Vorrichtung besonders zweckmäßig Teil einer Endoskopievorrichtung sein, wobei das selbstschmierende Element als Stülpschlauch ausgebildet ist.

Vorzugsweise ist es das selbstschmierende Element, welches mit einem menschlichen oder tierischen Körper in Eingriff bringbar ist und dabei mit diesem in Kontakt tritt. Da keine übermäßigen Schmierstoffmengen abgegeben werden, verhält sich das Element gegenüber der Umgebung, insbesondere gegenüber dem Körpergewebe im Wesentlichen neutral, so dass diese bevorzugte Ausgestaltung der Erfindung einen Kontakt zwischen Vorrichtung und Körpergewebe zulässt.

In einer konkreten, besonders bevorzugten Ausgestaltung bildet das selbstschmierende Element daher einen Stülpschlauch, insbesondere einen Stülpschlauch für ein Endoskop (z.B. ein Koloskop, ein Jejunoskop, ein Gastroskop oder ein Bronchoskop).

Die erfindungsgemäße Vorrichtung kann zusätzlich zu dem Element ein Gegenelement umfassen, wobei es, zusätzlich zu der Reibung zwischen unterschiedlichen Abschnitten des selbstschmierenden Elements, zu einer Reibung zwischen wenigstens einer Oberfläche des Elements und wenigstens einer Oberfläche des Gegenelements kommt.

Der Reibungskoeffizient zwischen den wenigstens zwei gegenüberliegenden Flächen wird durch die selbstschmierenden Eigenschaften des Elements beträchtlich verringert, was die Gleitfähigkeit von Element und Gegenelement gegeneinander stark verbessert.

Eine weitere Verbesserung dieser Gleitfähigkeit kann erzielt werden, indem das Gegenelement oder ein Mantel bzw. eine Umhüllung von diesem so ausgebildet ist, wie es vorstehend für das einer inneren Reibung ausgesetzte selbstschmierende Element beschrieben wurde. Hierbei kann für das Gegenelement jede der vorstehenden Ausführungsformen eingesetzt werden, wobei Element und Gegenelement in der medizintechnischen Vorrichtung abhängig von den Umständen der beabsichtigen Anwendung in gleicher oder unterschiedlicher Weise ausgestaltet sein können. Allerdings können das Gegenelement bzw. sein Mantel auch aus einem Material gebildet sein, welches keine selbstschmierenden Eigenschaften aufweist.

Eine vorteilhafte Ausführung besteht darin, dass die Oberfläche des Gegenelements ebenfalls mit einem Schmiermittel versehen ist. Dieses Schmiermittel kann ein anhaftendes Öl, ein auflaminierter Schmierfilm und/oder ein festes Schmiermittel sein.

Für das anhaftende Öl können dieselben Öle verwendet werden, wie sie vorstehend als Beispiele für das Schmiermittel angegeben wurden.

Der auflaminierte Schmierfilm kann beispielsweise aus den Kautschuken bestehen, die vorstehend als geeignete Materialien für den Werkstoff beschrieben wurden.

Das feste Schmiermittel, das vorteilhaft auf der Oberfläche des Gegenelements vorliegen kann, kann entweder direkt auf der Oberfläche aufgebracht sein oder kann mittels eines Bindeharzes an die Oberfläche angehaftet sein. Ein solches Bindeharz kann einen oder mehrere der vorstehend beschriebenen Kautschuke umfassen.

Konkrete, aufgrund ihrer guten Gleiteigenschaften bevorzugte Beispiele für das feste Schmiermittel schließen Kohlenstoffverbindungen wie Graphit, Nitride wie Bornitrid, fluorhaltige Verbindungen wie etwa Fluorharze und Sulfide wie etwa Molybdänsulfid und Wolframsulfid ein.

Das Gegenelement ist in einer bevorzugten Ausführungsform ein Endoskopschaft, der vorzugsweise in einen durch das selbstschmierende Element gebildeten Stülpschlauch eingeführt ist und dessen Oberfläche oder Ummantelung vorzugsweise wie ein selbstschmierendes Element gemäß der Erfindung ausgestaltet ist. Die Oberfläche bzw. die Ummantelung, welche selber als Schlauch vorliegt, kann aber selbstverständlich auch aus einem Material bestehen, welches keine selbstschmierenden Eigenschaften aufweist, solange das selbstschmierende Element der vorliegenden Erfindung als Stülpschlauch zum Einsatz kommt.

Es ist zudem vorteilhaft und bevorzugt, dass das erfindungsgemäß verwendete Schmiermittel einen Wirkstoff einschließt. Dabei kann ein Schmiermittel einen Wirkstoff ohne Schmiereigenschaften enthalten, oder der Wirkstoff hat selber Schmierwirkung und bildet teilweise oder vollständig das Schmiermittel. Auf diese Weise können in der medizintechnischen Anwendung jeweils zweckmäßige Wirkstoffe, in der Endoskopie insbesondere Schmerzmittel, blutstillende Mittel usw., lokal und zielgerichtet eingesetzt und zugeführt werden.

In der vorliegenden Erfindung sind insbesondere reizlindernde Mittel, entzündungshemmende Mittel, abschwellend wirkende Mittel, blutstillende Mittel, desinfizierende Mittel, Befeuchtungsmittel und Schmerzmittel bevorzugt, da diese bei einer Verwerdung der erfindungsgemäßen Vorrichtung im tierischen oder menschlichen Körper die jeweils spezifischen, vorteilhaften Wirkungen hervorrufen.

### BEISPIELE

### Beispiel 1

Ein handelsüblicher Siliconkautschuk wurde zu einem Stülpschlauch für ein Endoskop mit einer Länge von 3,20 cm extrudiert.

Mit einem CO₂-Laser mit einer Wellenlänge von 10600 nm (Pulsdauer 40 µs) wurden dann zur Mantellinie der Schläuche schräg verlaufende Nuten eingraviert. Dabei wurden Nuten mit einer Tiefe von 300 µm ausgebildet.

Die Nuten wurden bei 60°C mit bei dieser Temperatur flüssigem Palmfett befüllt (Starttemperatur der Erweichung des Fetts: >35°C). Anschließend ließ man den Schlauch abkühlen, wobei sich das Palmfett in den Nuten verfestigte.

Der resultierende Schlauch wurde als ein Stülpschlauch für ein Endoskop verwendet. Es zeigte sich, dass die Reibung zwischen Schlauch und Endoskopschaft sowie zwischen den Schlauchabschnitten im Umstülpbereich deutlich herabgesetzt war, eine verbesserte Schmierwirkung eintrat und gleichzeitig kaum Schmiermittel aus der Endoskopvorrichtung austrat.

### Beispiel 2

Zwei Schläuche wurden wie im Beispiel 1 extrudiert, von denen einer wie im Beispiel 1 mit dem Laser graviert wurde. Anschließend wurden beide Schläuche mit einem Keramikfilm beschichtet. Die Nuten des gravierten Schlauches wurden wie im Beispiel 1 mit Palmfett befüllt. Auf diese Weise wurden in beiden Fällen hervorragende Gleiteigenschaften erzielt.

### Beispiel 3

Zwei Schläuche wurden wie im Beispiel 1 extrudiert, von denen einer wie im Beispiel 1 mit dem Laser graviert wurde. Anschließend wurden beide Schläuche an der Oberfläche gemäß einem Niederdruck-Plasmaverfahren silicatisiert. Die Nuten des gravierten Schlauches wurden wie im Beispiel 1 mit Palmfett befüllt. Auf diese Weise wurden in beiden Fällen hervorragende Gleiteigenschaften erzielt.

### Beispiel 4

Zwei Schläuche wurden wie im Beispiel 1 extrudiert, von denen einer wie im Beispiel 1 mit dem Laser graviert wurde. Anschließend wurden beide Schläuche in einer Stickstoffatmosphäre für 10 Minuten mit UV-Licht bestrahlt. Die Nuten des gravierten Schlauches wurden wie im Beispiel 1 mit Palmfett befüllt. Auf diese Weise wurden in beiden Fällen hervorragende Gleiteigenschaften erzielt.

### Beispiel 5

Zwei Schläuche wurden wie im Beispiel 1 extrudiert, von denen einer wie im Beispiel 1 mit dem Laser graviert wurde. Dann wurden beide Schläuche durch Corona-Aktivierung vorbehandelt. Anschließend wurden die Schläuche durch Tauchbeschichten mit einer Acrylatschicht beschichtet. Die Nuten des gravierten Schlauches wurden wie im Beispiel 1 mit Palmfett befüllt. Auf diese Weise wurden in beiden Fällen hervorragende Gleiteigenschaften erzielt.

### Beispiel 6

Das Beispiel 5 wurde wiederholt, mit der Ausnahme, dass anstelle der Corona-Vorbehandlung eine Fluorierung als Vorbehandlung erfolgte. Die Ergebnisse waren im Wesentlichen ebenso gut wie jene, die im Beispiel 5 erzielt wurden.

### Beispiel 7

Das Beispiel 5 wurde wiederholt, mit der Ausnahme, dass anstelle der Corona-Vorbehandlung ein manuelles Beflammen als Vorbehandlung erfolgte. Die Ergebnisse waren im Wesentlichen ebenso gut wie jene, die im Beispiel 5 erzielt wurden.

### Beispiel 8

Zwei Schläuche wurden wie im Beispiel 1 extrudiert, von denen einer wie im Beispiel 1 mit dem Laser graviert wurde. Anschließend wurden beide Schläuche durch Tauchbeschichten mit einer Perhydropolysilazanmischung beschichtet. Die Beschichtung wurde bei erhöhter Temperatur oxidativ in eine Polysilazanschicht umgewandelt. Die Nuten des gravierten Schlauches wurden wie im Beispiel 1 mit Palmfett befüllt. Auf diese Weise wurden in beiden Fällen hervorragende Gleiteigenschaften erzielt.

### Beispiel 9

Die Versuche der Beispiele 1 bis 8, bei denen der Laser zum Einsatz kam, wurden wiederholt, mit der Ausnahme, dass ein Schlauch mit einer Länge von 3,50 m eingesetzt wurde, und wobei diesmal keine Nuten, sondern im wesentlichen kreisförmige Kavitäten mit einer Tiefe von 100 bis 150 µm und einem Durchmesser von 150 µm erzeugt wurden. Diese Kavitäten wurden mit Palmfett befüllt.

Die so hergestellten Stülpschläuche wurden bei endoskopische Untersuchungen des Dick- und Dünndarms eingesetzt. Es zeigte sich, dass auch bei tieferer Einführung in das untersuchte Subjekt eine konstant gute Schmierung beibehalten werden konnte.

### Beispiel 10

Der im Beispiel 1 eingesetzte Siliconkautschuk wurde zu acht Stülpschlauchen mit jeweils einer Länge von 3,20 cm extrudiert.

Anschließend wurden vier Schläuche nacheinander in eine Reaktionskammer (Vakuum < 0,5 mbar) für eine Plasmabehandlung eingesetzt. Unter Verwendung von O₂ als Plasmagas wurde für einige Sekunden eine Plasmavorbehandlung so durchgeführt, dass die Oberfläche des Siliconschlauches auf Mikroebene aufgeraut wurde.

In einem zweiten Schritt wurden sechs Schläuche (drei mit Vorbehandlung, drei ohne) einer Niederdruckplasmabehandlung unterzogen (Vakuum < 0,5 mbar; Temperatur = 20-40°C), um eine Beschichtung auszubilden. Auf diese Weise wurden jeweils zwei Schläuche mit Siloxan, mit Fluorcarbon bzw. mit ePTFE beschichtet.

Alle acht Schläuche wurden als Stülpschläuche für einen Endoskopschaft eingesetzt, und die Gleiteigenschaften wurden gemäß den folgenden Kriterien bewertet.
A: Hervorragende Gleiteigenschaften;
B: sehr gute Gleiteigenschaften;
C: noch überdurchschnittliche Gleiteigenschaften;
D: zu starke Reibung.

Die nachstehende Tabelle 1 gibt die Ergebnisse der Bewertung wieder:

**Tabelle 1**

| Schlauch Nr. | Plasmavorbehandlung | Beschichtung | Gleiteigenschaft |
|---|---|---|---|
| 1 | Ja | - | C |
| 2 | Nein | Siloxan | B |
| 3 | Ja | Siloxan | A |
| 4 | Nein | Fluorcarbon | B |
| 5 | Ja | Fluorcarbon | A |
| 6 | Nein | ePTFE | B |
| 7 | Ja | ePTFE | A |
| 8 | Nein | - | D |

### Beispiel 11

Es wurden erneut Schläuche 1 bis 7 gemäß Beispiel 10 hergestellt, wie im Beispiel 9 durch Lasergravur mit Kavitäten versehen und anschließend wie im Beispiel 10 ggf. plasmavorbehandelt (Schläuche 1, 3, 5 und 7) und dann plasmabeschichtet (nicht Schlauch 1). Die Kavitäten wurden mit Palmfett befüllt. Die Bewertung wurde analog zum Beispiel 10 durchgeführt und ergab vergleichbar gute Ergebnisse.

## Patentansprüche

1. Mit einem menschlichen oder tierischen Körper in Eingriff bringbare medizintechnische Vorrichtung in Form eines Endoskops mit einer inneren Reibung ausgesetzten selbstschmierenden Elementen in Form eines Endoskopschafts und eines Stülpschlauchs, jeweils bestehend aus einem Material, das ursprünglich keine selbstschmierenden Eigenschaften hat und das durch eine Oberflächenbehandlung so modifiziert ist, dass dieses zu einem selbstschmierenden Element wird, **dadurch gekennzeichnet, daß** zum Einen die Oberfläche des Endoskopschafts Kavitäten aufweist, die ein Schmiermittel enthalten und zum Anderen das Stülpschlauchmaterial Silikonkautschuk aufweist oder aus Silikonkautschuk besteht sowie eine Oberflächenschicht hat, welche ein Schmiermittel vorzugsweise aus einem Material enthält ausgewählt aus: Graphit, Nitrid, Fluorkohlenwasserstoff, MoS₂, WS₂, Talk, Öl, Polysilazan, Polysiloxan, PTFE, ePTFE, Gelatine, Agar-Agar, Cellulose, eine Kombination aus Polysaccharid und einem Protein oder Silikonöl.

2. Vorrichtung nach Anspruch 1, wobei es das selbstschmierende Element selber ist, welches mit einem menschlichen oder tierischen Körper in Eingriff bringbar ist und mit dem Körper in Kontakt tritt.

3. Vorrichtung nach Anspruch 2, wobei mindestes eine Oberfläche des selbstschmierenden Elements in Form des Endoskopschafts plasmabehandelt ist.

4. Vorrichtung nach Anspruch 3, wobei die plasmabehandelte Oberfläche plasmabeschichtet ist.

5. Vorrichtung nach Anspruch 4, wobei die plasmabeschichtete Oberfläche mit Siloxan, Fluorcarbon, PTFE, ePTFE und/oder Polyurethan beschichtet ist.

6. Vorrichtung nach einem der Ansprüche 1 bis 5, wobei die Oberflache des selbstschmierenden Elements in Form des Endoskopschafts und/oder des Stülpschlauchs hydrophil ist.

7. Vorrichtung nach einem der Ansprüche 1 bis 5, wobei die Oberflache des selbstschmierenden Elements in Form des Endoskopschafts und/oder des Stülpschlauchs hydrophob ist.

8. Vorrichtung nach Anspruch 1, wobei die Kavitäten durch Gravieren mit einem Laser erzeugbar sind.

9. Vorrichtung nach Anspruch 1 oder8, wobei über den Kavitäten eine Plasmabeschichtung ausgebildet ist.

10. Vorrichtung nach einem der Ansprüche 1 und 8 bis 9, wobei die Kavitäten so angepasst sind, dass freigesetztes Schmiermittel zu Bereichen des selbstschmierenden Elements in Form des Endoskopschafts zugeführt wird, welche einer größeren Reibung ausgesetzt sind als andere Bereiche.

11. Vorrichtung nach Anspruch1, wobei mindestens eine Oberfläche des selbstschmierenden Elements in Form des Endoskopschafts mit Siloxan, Fluorcarbon, PTFE, ePTFE und/oder Polyurethan beschichtet ist.

12. Vorrichtung nach Anspruch1, wobei mindestens eine Oberfläche des Stülpschlauchs mit Siloxan, Fluorcarbon, PTFE, ePTFE und/oder Polyurethan beschichtet ist.

## Claims

1. A medico-technical apparatus adapted to be engaged with a human or animal body, having the form of an endoscope comprising self-lubricating elements which are subjected to an internal friction and have the form of an endoscope shaft and an everting tube, each of which consists of a material that originally lacks self-lubricating properties and is modified through a surface treatment so as to become a self-lubricating element, **characterized in that** on the one hand the surface of the endoscope shaft includes cavities which contain a lubricant and on the other hand in that the everting tube material includes silicone rubber or consists of silicone rubber, and has a surface layer containing a lubricant preferably of a material that is selected from: graphite, nitride, fluorohydrocarbon, MoS₂, WS₂, talc, oil, polysilazane, polysiloxane, PTFE, ePTFE, gelatin, agar-agar, cellulose, a combination of polysaccharide and a protein or silicone oil.

2. The apparatus according to claim 1, wherein it is the very self-lubricating element that is adapted to be engaged with a human or animal body and enters into contact with said body.

3. The apparatus according to claim 2, wherein at least one surface of the self-lubricating element having the form of the endoscope shaft has been plasma-treated.

4. The apparatus according to claim 3, wherein the plasma-treated surface is plasma-coated.

5. The apparatus according to claim 4, wherein the plasma-coated surface is coated with siloxane, fluorocarbon, PTFE, ePTFE and/or polyurethane.

6. The apparatus according to any one of claims 1 to 5, wherein the surface of the self-lubricating element having the form of the endoscope shaft and/or the everting tube is hydrophilic.

7. The apparatus according to any one of claims 1 to 5, wherein the surface of the self-lubricating element having the form of the endoscope shaft and/or the everting tube is hydrophobic.

8. The apparatus according to claim 1, wherein the cavities may be created through engraving by a laser.

9. The apparatus according to claim 1 or 8, wherein a plasma coating is formed over the cavities.

10. The apparatus according to any one of claims 1 and 8 to 9, wherein the cavities are adapted such that released lubricant is supplied to regions of the self-lubricating element having the form of the endoscope shaft that are subjected to higher friction than other regions.

11. The apparatus according to claim 1, wherein at least one surface of the self-lubricating element having the form of the endoscope shaft is coated with siloxane, fluorocarbon, PTFE, ePTFE and/or polyurethane.

12. The apparatus according to claim 1, wherein at least one surface of the everting tube is coated with siloxane, fluorocarbon, PTFE, ePTFE and/or polyurethane.

## Revendications

1. Dispositif médical, pouvant être mis en prise avec un corps humain ou animal, sous forme d'un endoscope, comportant un élément autolubrifiant interne, exposé au frottement, sous forme d'une tige d'endoscope et d'un tuyau flexible inversé, chacun étant constitué d'un matériau qui initialement n'a aucune propriété autolubrifiante, et qui grâce à un traitement de surface est modifié de façon qu'il devienne un élément autolubrifiant, **caractérisé en ce que** d'une part la surface de la tige de l'endoscope comprend des cavités qui contiennent un lubrifiant, et d'autre part le matériau du tuyau flexible inversé comprend un caoutchouc silicone ou est constitué d'un caoutchouc silicone, et possède une couche superficielle qui contient un lubrifiant, de préférence en un matériau choisi parmi le graphite, un nitrure, un hydrocarbure fluoré, MoS₂, WS₂, le talc, l'huile, un polysilazane, un polysiloxane, le PTFE, l'ePTFE, la gélatine, l'agar-agar, la cellulose, ou une combinaison d'un polysaccharide et d'une protéine ou d'une huile de silicone.

2. Dispositif selon la revendication 1, qui est par lui-même l'élément autolubrifiant, qui peut être mis en prise avec un corps humain ou animal et qui entre en contact avec le corps.

3. Dispositif selon la revendication 2, dans lequel au moins une surface de l'élément autolubrifiant sous forme d'une tige d'endoscope est traitée par plasma.

4. Dispositif selon la revendication 3, dans lequel la surface traitée par plasma est revêtue par plasma.

5. Dispositif selon la revendication 4, dans lequel la surface revêtue par plasma est revêtue de siloxane, d'un fluorocarbure, de PTFE, d'ePTFE et/ou de polyuréthanne.

6. Dispositif selon l'une des revendications 1 à 5, dans lequel la surface de l'élément autolubrifiant, sous forme de la tige d'endoscope et/ou du tuyau flexible inversé, est hydrophile.

7. Dispositif selon l'une des revendications 1 à 5, dans lequel la surface de l'élément autolubrifiant sous forme de la tige d'endoscope et/ou du tuyau flexible inversé est hydrophobe.

8. Dispositif selon la revendication 1, dans lequel les cavités peuvent être obtenues par gravure par un laser.

9. Dispositif selon la revendication 1 ou 8, dans lequel un revêtement par plasma est réalisé par-dessus les cavités.

10. Dispositif selon l'une des revendications 1 et 8 à 9, dans lequel les cavités sont ajustées de telle sorte que le lubrifiant libéré soit envoyé dans des zones de l'élément autolubrifiant sous forme de la tige d'endoscope qui sont exposées à un frottement plus grand que les autres zones.

11. Dispositif selon la revendication 1, dans lequel au moins une surface de l'élément autolubrifiant sous forme de la tige d'endoscope est revêtue d'un siloxane, d'un fluorocarbure, de PTFE, d'ePTFE et/ou de polyuréthanne.

12. Dispositif selon la revendication 1, dans lequel au moins une surface du tuyau flexible inversé est revêtue d'un siloxane, d'un fluorocarbure, de PTFE, d'ePTFE et/ou de polyuréthanne.
